# EUROPEAN PATENT APPLICATION

(11) **EP 4 772 610 A1**
(43) Date of publication of application: **08.07.2026**
(21) Application number: 24860471.2
(22) Date of filing: 30.08.2024
(51) Int. Cl.: C12N 1/20, A61K 35/747, A61P 3/04, A61P 3/10, A23L 33/135, A23K 10/18, C12R 1/25

(54) **LACTIPLANTIBACILLUS PLANTARUM STRAIN AND COMPOSITION FOR PREVENTING OR TREATING METABOLIC DISEASES COMPRISING SAME**

(30) Priority: 31.08.2023 KR 20230115572; 08.12.2023 KR 20230177308; 26.02.2024 KR 20240026979; 04.04.2024 KR 20240045777
(71) Applicant: GI Longevity Inc., Seongnam-si, Gyeonggi-do 13201 (KR)
(72) Inventor: YANG, Bo Gie, Seoul Seoul 05849 (KR); JANG, Myung Ho, Seoul Seoul 05849 (KR); KIM, Jin Wook, Seongnam-si, Gyeonggi-do Gyeonggi-do 13356 (KR)
(74) Representative: Habermann, Hruschka & Schnabel
(86) International application number: PCT/KR2024/013073
(87) International publication number: WO 2025/048554

(57) **Abstract**

Disclosed herein is a novel *Lactiplantibacillus plantarum* strain possessing a DPP-4 activity inhibitory effect and a beneficial metabolite secretory effect and being capable of stimulating GLP-1 secretion. Also provided herein is a pharmaceutical composition for treating or preventing metabolic diseases, comprising said strain or a culture thereof.

## Description

### FIELD OF THE INVENTION

The present disclosure pertains to a *Lactiplantibacillus plantarum* strain. Furthermore, the present disclosure also pertains to pharmaceutical compositions for treating or preventing metabolic diseases comprising this strain.

### BACKGROUND OF THE INVENTION

Probiotics refer to microorganisms with antimicrobial and enzymatic activities that can help balance intestinal microorganisms. Probiotics are defined as live cells consisting of single or multiple strains that improve intestinal microflora when administered to humans or animals in the form of dried cells or fermentation products. The requisite characteristics for probiotics are ability to take up residence in the human intestines, non-pathogenicity, nontoxicity and ability to survive their passage to the intestines. Furthermore, they must maintain viability and activity until consumption within the food to be delivered, be sensitive to antibiotics used for preventing infection, and not harbor antibiotic resistance plasmids. Moreover, they must have resistance to acids, enzymes and bile in the intestinal environment. Recent reports of their various health-enhancing effects, including improving intestinal health probiotics have led to their emergence as prominent therapeutic agents that may replace existing compound-based treatments.

Meanwhile, among the various metabolites secreted by commensal microbiota such as lactic acid bacteria, during their life processes, some are known to exert metabolically beneficial effects on host cells. For example, 3-phenyllactic acid is known to regulate intestinal lipid metabolism, thereby exerting an effect in preventing obesity or type 2 diabetes in the host. α-ketoglutarate, an intermediate in the Krebs cycle, has been reported to provide such improvements in lipid metabolism as limiting host weight gain, suppressing inflammation, and promoting adipocyte browning through regulation of the gut microflora in which the proportion of bacteria belonging to the phylum Bacteroidetes increases while that of bacteria belonging to the phylum Firmicutes decreases.

Regarding probiotic lactic acid bacteria associated with such effects on prevention and treatment of diabetes, Korean Registered Patent No. 10-1494279 discloses the *Lactobacillus plantarum* strain KY1032 (Accession No.: KCCM-10430) possessing efficacy in inhibiting adipocyte differentiation, Korean Registered Patent No. 10-0996577 discloses *Lactobacillus curvatus* HY7601 (Accession No.: KCTC 11456BP) with obesity-suppressing activity, and Korean Registered Patent No. 10-1394348 discloses the *Lactobacillus plantarum* strain DSR920 (Accession No.: KCCM 11210P) possessing efficacy in inhibiting adipocyte differentiation. However, their efficacies have not yet reached a level sufficient for commercial success.

### TECHNICAL PROBLEM

The technical objective of the present invention is to develop a novel lactic acid bacterial strain with excellent efficacy in the prevention and treatment of metabolic diseases.

### SUMMARY OF THE INVENTION

To achieve the above objective, in one aspect of the present invention is provided the *Lactiplantibacillus plantarum* strain GBCC_F0227 (Accession No.: KCTC 15450BP).

In another aspect of the present invention is provided a pharmaceutical composition for the prevention or treatment of a metabolic disease comprising as an active ingredient the *Lactiplantibacillus plantarum* strain GBCC_F0227 (Accession No.: KCTC 15450BP) or a culture thereof.

In still another aspect of the present invention is provided a food composition comprising the *Lactiplantibacillus plantarum* strain GBCC_F0227 (Accession No.: KCTC 15450BP) or a culture thereof.

In yet another aspect of the present invention, a dietary supplement comprising the *Lactibacillus plantarum* strain GBCC_F0227 (Accession No.: KCTC 15450BP) or a culture thereof is provided.

In still yet another aspect of the present invention, a feed composition comprising the *Lactiplantibacillus plantarum* GBCC_F0227 strain (Accession No.: KCTC 15450BP) or a culture thereof is provided.

In a still further aspect of the present invention is provided a pharmaceutical composition for the treatment or prevention of a metabolic disease that stimulates the host GLP-1 secretion or suppresses appetite in a host, comprising as an active ingredient at least one selected from the group consisting of a strain of the species *Lactiplantibacillus plantarum,* a lysate of the strain, a cell extract of the strain, killed cells of the strain, a culture of the strain, an extract of the culture of the strain, and a cell-free culture supernatant of the strain.

### ADVANTAGEOUS EFFECTS

The *Lactiplantibacillus plantarum* GBCC_F0227 strain of the present invention (Accession No.: KCTC 15450BP) has an excellent GLP-1 secretion stimulation efficacy, including the capability to secrete outside the cell a large amount of metabolites effective in stimulating host GLP-1 secretion. Furthermore, it is capable of effectively inhibiting DPP-4 enzyme activity as well as promoting the expression of GLP-1 receptors, thereby doubling the beneficial effects of GLP-1 action. Strain GBCC_F0227 of the present invention is effective in improving lipid metabolism. In particular, it reduces white fat accumulation that may accompany obesity in visceral adipose tissues such as the liver and restores adiponectin secretion, and is also able to suppress the expression of such genes as CD36 and PPARγ that contribute to visceral triglyceride accumulation. Therefore, strain GBCC_F0227 and a composition comprising thereof can contribute to the prevention and treatment of fatty liver and non-alcoholic fatty liver disease. In addition, strain GBCC_F0227 of the present invention also exhibits excellent blood glucose-improving effects, and importantly, since it can reduce fasting blood glucose levels, it can effectively improve insulin resistance by ameliorating glucose tolerance and augmenting insulin sensitivity. Furthermore, strain GBCC_F0227 of the present invention possesses efficacy in preventing increases in blood triglycerides and blood cholesterol associated with diet-induced obesity. Moreover, strain GBCC_F0227 of the present invention can secrete a large amount of metabolites that suppress weight gain, and on top of this enhanced GLP-1 secretion, it can also promote gene expression of GLP-1 receptor, POMC, and CART, which contribute to suppressing appetite under obese conditions. Consequently, it exhibits excellent anti-obesity effects in which weight gain is suppressed and body fat composition is improved. Strain GBCC_F0227 of the present invention and compositions comprising thereof can thus be usefully employed in preventing or treating metabolic diseases such as obesity and metabolic syndrome.

### BRIEF DESCRIPTION OF THE FIGURES

Figure 1a is a graph evaluating the extent to which the culture supernatant of *Lactiplantibacillus plantarum* strains, a species of lactic acid bacteria, isolated from fermented foods, stimulates GLP-1 secretion in NCI-H716 cells. In the graph, NS denotes the control condition where no sample was added to the NCI-H716 cells, while MRS indicates a treatment with culture media that has not been used for culture.
Figure 1b is a graph evaluating the extent to which the culture supernatant of *Lactiplantibacillus paracasei* strains, a species of lactic acid bacteria, isolated from fermented foods, stimulates GLP-1 secretion in NCI-H716 cells. In the graph, NS denotes the control condition where no sample was added to the NCI-H716 cells, while MRS indicates a treatment with culture media that has not been used for culture.
Figure 1c is a graph evaluating the extent to which the culture supernatant of *Lactiplantibacillus corniniformis* strains, a species of lactic acid bacteria, isolated from fermented foods, stimulates GLP-1 secretion in NCI-H716 cells. In the graph, NS denotes the control condition where no sample was added to the NCI-H716 cell line, while MRS indicates a treatment with culture media that has not been used for culture.
Figure 1d is a graph evaluating the extent to which the culture supernatant of *Lactiplantibacillus sakei* strains, a species of lactic acid bacteria, isolated from fermented foods, stimulates GLP-1 secretion in NCI-H716 cells. In the graph, NS denotes the control condition where no sample was added to the NCI-H716 cell line, while MRS indicates a treatment with culture media that has not been used for culture.
Figure 1e is a graph evaluating the extent to which the culture supernatant of *Lactiplantibacillus fermentum* strains, a species of lactic acid bacteria, isolated from fermented foods, stimulates GLP-1 secretion in NCI-H716 cells. In the graph, NS denotes the control condition where no sample was added to the NCI-H716 cell line, while MRS indicates a treatment with culture media that has not been used for culture.
Figure 1f is a graph evaluating the extent to which the culture supernatant from those isolates that were among the lactic acid bacteria strains isolated from fermented foods and belonging to the genus *Lactobacillus* under the old classification, but not to those species referred to above, stimulates GLP-1 secretion in NCI-H716 cells. In the graph, NS denotes the control condition where no sample was added to the NCI-H716 cells, while MRS indicates a treatment with culture media that has not been used for culture.
Figure 1g is a graph evaluating the extent to which the culture supernatant from those strains of genus *Bifidobacterium* among the lactic acid bacteria isolates from fermented foods, stimulates GLP-1 secretion in NCI-H716 cells. In the graph, NS denotes the control condition where no sample was added to the NCI-H716 cells, while MRS indicates a treatment with culture media that has not been used for culture.
Figure 1h is a phylogenetic tree constructed by comparing the GBCC_F0227 genome sequence with the genomes from other strains of *Lactiplantibacillus plantarum* and that of *Lactiplantibacillus argentoratensis.*
Figure 2a is a graph comparing the GLP-1 secretion-stimulating activity of *Lactiplantibacillus plantarum* GBCC_F0227 with that of a known commercially available strain of the same species as well as those of other strains of the same species isolated from fermented foods so as to determine whether this activity is specific to this strain. In the graph, NS denotes the control condition where no sample was added to the NCI-H716 cells while MRS indicates a treatment with culture media that has not been used for culture.
Figure 2b is a graph comparing the GLP-1 secretion-stimulating activities of *Lactiplantibacillus plantarum* isolates, including GBCC_F0227, with that of the known *plantarum* strain GB104.
Figure 3 is a graph showing the relationship between administration of strain GBCC_F0227 and blood GLP-1 levels in mice.
Figure 4a is a graph showing inhibitory efficacy of the selected *Lactiplantibacillus plantarum* strains against DPP-4 enzyme activity.
Figure 4b is a graph showing the experimental results comparing the DPP-4 enzyme activity inhibitory effects of the selected *Lactiplantibacillus plantarum* strains with that of a known *Lactiplantibacillus plantarum* strain.
Figure 4c is a graph showing the experimental results comparing the DPP-4 enzyme activity inhibitory effects of the strain GBCC_F0227 with strains of other species within the genus *Lactiplantibacillus.*
Figure 5 is a graph showing the correlation between GLP-1 secretion stimulation and DPP-4 activity inhibition for each selected and isolated strain of the present invention.
Figure 6a is a graph showing the extent to which strain GBCC_F0227 suppresses weight gain in mice fed a high fat diet.
Figure 6b is a graph illustrating the changes in the composition of fat mass and fatfree mass brought about by administration of strain GBCC_F0227 in diet-induced obese mice fed a high fat diet.
Figure 7a shows the results of an oral glucose tolerance test in mice administered with strain GBCC_F0227, Figure 7b shows fasting blood glucose measurements and Figure 7c shows the area under the curve for the graph of blood glucose versus time.
Figures 8a and 8b are tables showing the results of quantitative analysis of metabolites secreted by the *L. platanrum* strain of the present invention (indicated as F0227) and other lactic acid bacterial strains (*L. platanrum* strains GBCC_F0053 (indicated as F0053), GBCC_F0058 (indicated as F0058) and KCTC3108; *Limosilactobacillus fermentum* strain (GB102, KCTC3112); and *Lactobacillus paragasseri* strain (H0213, KCTC3163)) after cultivation in MRS medium.
Figure 9a is a graph of *in vitro* experimental results evaluating the efficacy of seven lactic acid bacteria metabolites in stimulating GLP-1 secretion from NCI-H716 cells in a dose-dependent manner.
Figure 9b is a graph quantifying the GLP-1 secreted by NCI-H716 cells in response to seven lactic acid bacteria metabolites at a concentration of 25 mM.
Figure 10a shows microscopic images of sections of the left lateral lobe in the liver and epididymal fat from the obese negative control mice (G1) fed a normal chow diet (NCD) and administered with cryoprotectant daily for 8 weeks, (G1), the obese positive control mice (G2) fed a high fat diet and administered cryoprotectant, or the experimental mice (G3) fed a high fat diet with concurrent administration of *Lactiplantibacillus plantarum* GBCC_F0227. The scale bars indicate 1000 µm in the top row of images, 500 µm in the bottom row, and 100 µm in the row below that.
Figure 10b is a graph showing the area fraction (%) of lipid droplets in adipocytes within the hepatic left lateral lobe tissue or the mean size (µm²) of lipid droplets in epididymal fat for the G1, G2, and G3 groups of mice in Figure 10a.
Figure 10c is a micrograph showing tissue sections from the left lateral lobe of the liver stained with Oil Red O for the mice in groups G1, G2, and G3 shown in Figure 10a. The scale bar in the images represents 100 µm.
Figure 10d is a graph showing the Oil Red O staining intensity measured in the experiment of Figure 10c as a percentage relative to the staining results of the control group (G1). In the graph, the * symbol indicates the significance level for the *p*-value for the corresponding measurement (*p**: <0.05, *p**:* <0.01, *p***:* <0.001) calculated using a one-way ANOVA Tukey test. Figure 10e is a graph showing that strain GBCC_F0227 inhibits increases in blood triglyceride and cholesterol levels induced by a high fat diet in a mouse model.
Figure 11 shows a graph measuring the triglyceride concentrations in the culture supernatant before and after incubation for 31 probiotic strains, including *Lactiplantibacillus plantarum* GBCC_F0227.
Figure 12 is a graph showing the triglyceride concentration in each of the supernatants obtained by culturing five strains of the *L. plantarum* species, including *Lactiplantibacillus plantarum* GBCC_F0227, until the absorbance OD₆₀₀ at 600 nm reached 3 or 6 (Figure 12a) and a graph organizing the results based on the absorbance values (Figure 12b). In the graph of Figure 12, the *** symbol indicates that the p value calculated by performing a one-way ANOVA test on the corresponding triglyceride measurement value is less than 0.001.

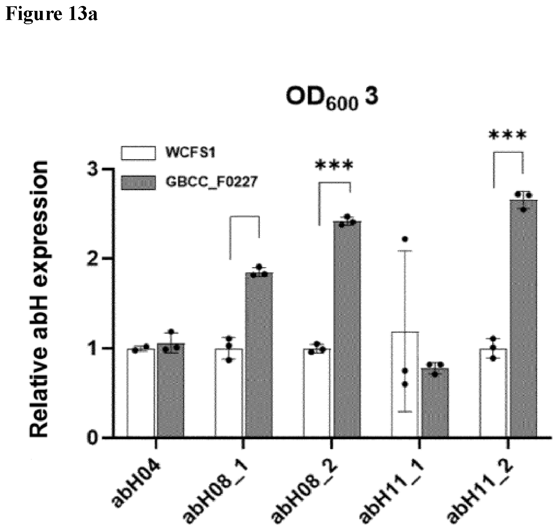

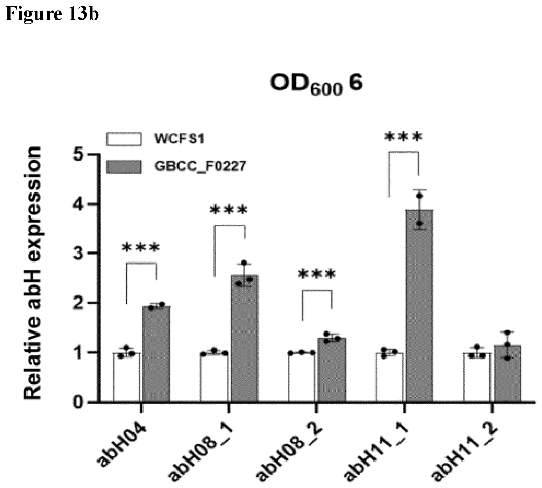

Figure 13 shows a graph measuring the relative expression levels of genes belonging to the α/β hydrolase gene family when *Lactiplantibacillus plantarum* GBCC_F0227 and *Lactiplantibacillus plantarum* WCFS1 strains were cultured until their optical density at 600 nm (OD600) reached 3 (Figure 13a) or 6 (Figure 13b). In the graphs of Figure 13, the ** symbol indicates that the *p*-value calculated using the unpaired Student's *t*-test on the measured relative expression levels for the corresponding gene is less than 0.01, while the *** symbol indicates that the calculated *p*-value is less than 0.001.

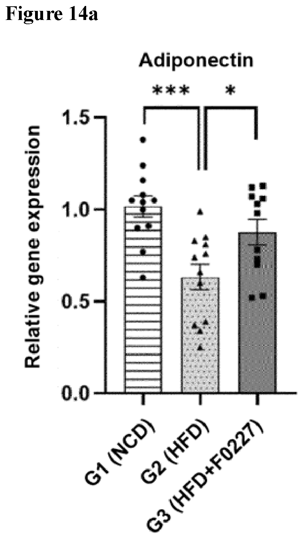

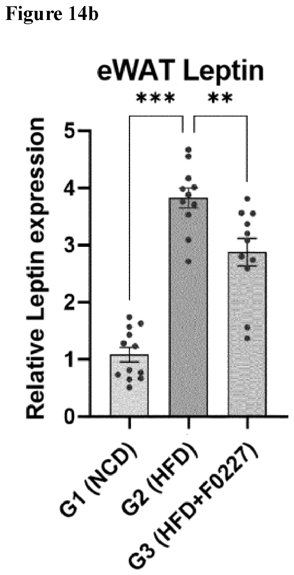

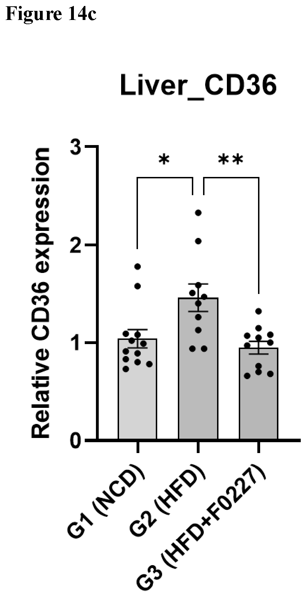

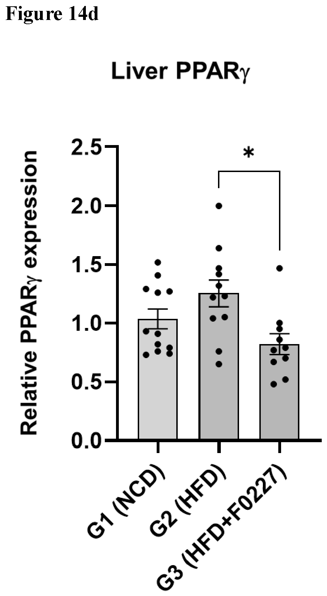

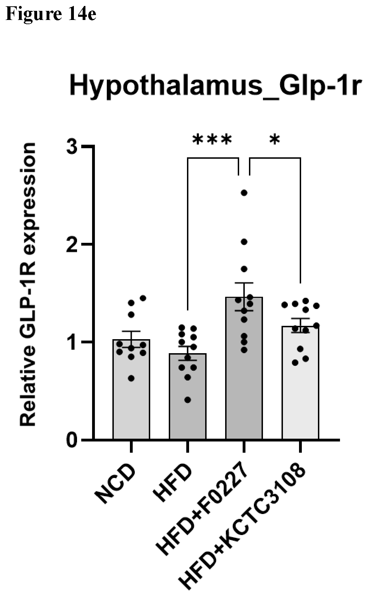

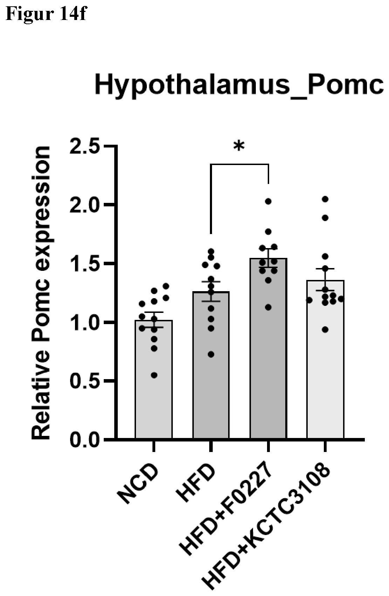

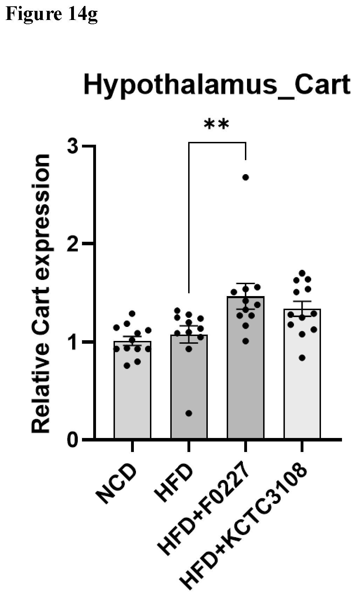

Figure 14 shows the measurement results of the expression levels of genes related to fat metabolism and obesity for three groups of mice consisting of obese negative control group (G1) fed a normal chow diet (NCD) and a cryoprotectant daily for 8 weeks, obese positive control group (G2) fed a high fat diet and a cryoprotectant daily and experimental group (G3) fed a high fat diet with concurrent administration of GBCC_F0227, or alternatively, in the case of Figures 14e to 14g, four groups of control mice (G4) administered a high fat diet and the same amount of KCTC 3108 strain as G3. In the graphs of Figure 14, the * symbol indicates that the *p*-value obtained from a one-way ANOVA test on the corresponding measured value compared to the reference measured value is less than 0.05; the ** symbol indicates that the *p*-value is less than 0.01; and the *** symbol indicates that the p-value is less than 0.001.
The changes in the expression levels are shown for the adiponectin gene in mouse epididymis (Figure 14a), the leptin gene in mouse epididymis (Figure 14b), the CD36 gene in the left hepatic lobe (Figure 14c), the PPARγ gene in the hepatic left lateral lobe (Figure 14d), the GLP-1 receptor gene in the hypothalamus (Figure 14e), the POMC gene in the hypothalamus (Figure 14f) and the CART gene in the hypothalamus (Figure 14g), respectively.

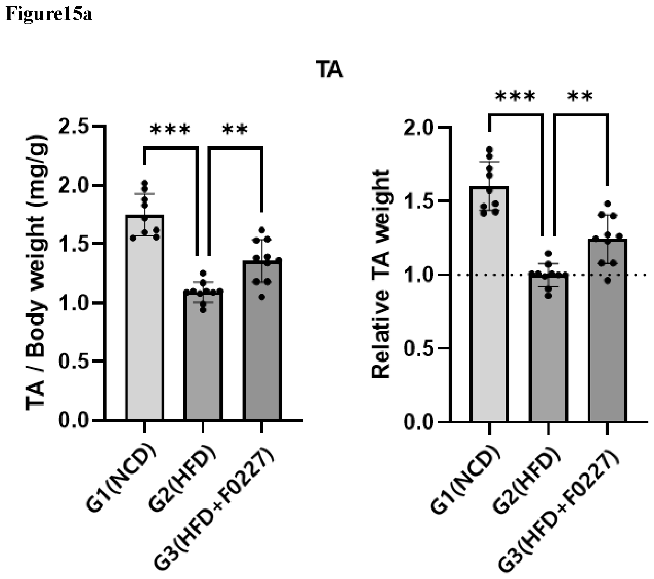

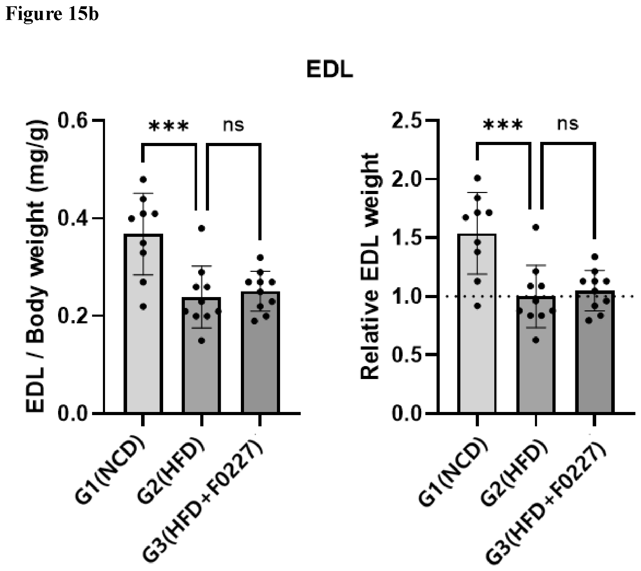

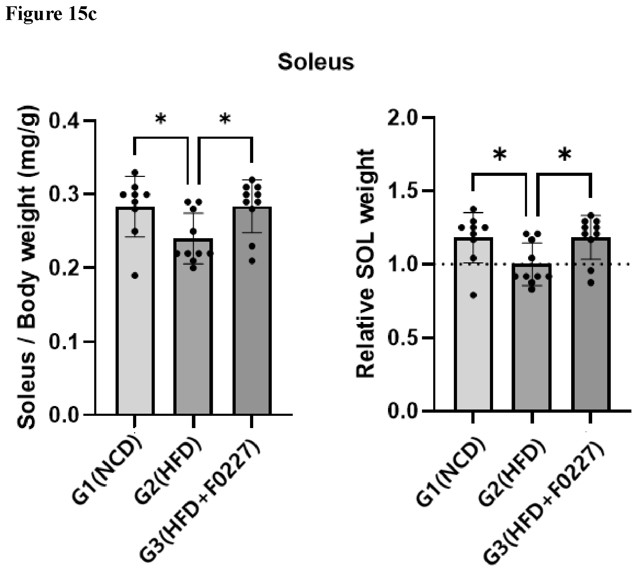

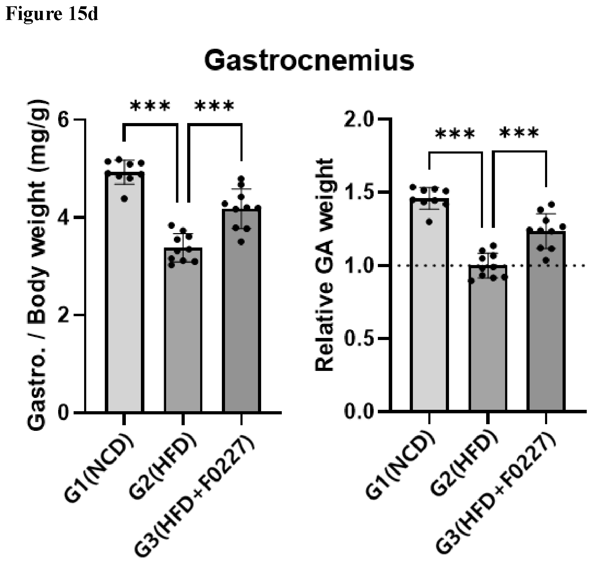

Figure 15 is a graph showing the change in hindlimb skeletal muscle mass in three groups of mice: the obese negative control mice (G1) that were administered a normal chow diet (NCD) and cryoprotectant daily for 8 weeks, the obese positive control mice (G2) that were administered a high fat diet and cryoprotectant, or the experimental group mice (G3) that were co-administered with a high fat diet and *Lactobacillus plantarum* GBCC_F0227. In the graph of Figure 15, the * symbol indicates that the *p*-value calculated by performing a one-way analysis of variance test on the corresponding measurement value compared to the reference measurement value is less than 0.05; the ** symbol indicates that the *p*-value is less than 0.01; the *** symbol indicates that the *p*-value is less than 0.001; and ns indicates a statistically non-significant difference. Muscle mass is shown in the graph of Figure 15a for the tibialis anterior (TA), Figure 15b for extensor digitorum longus (EDL), Figure 15c for the soleus (Sol), and Figure 15d for gastrocnemius muscle (GA).

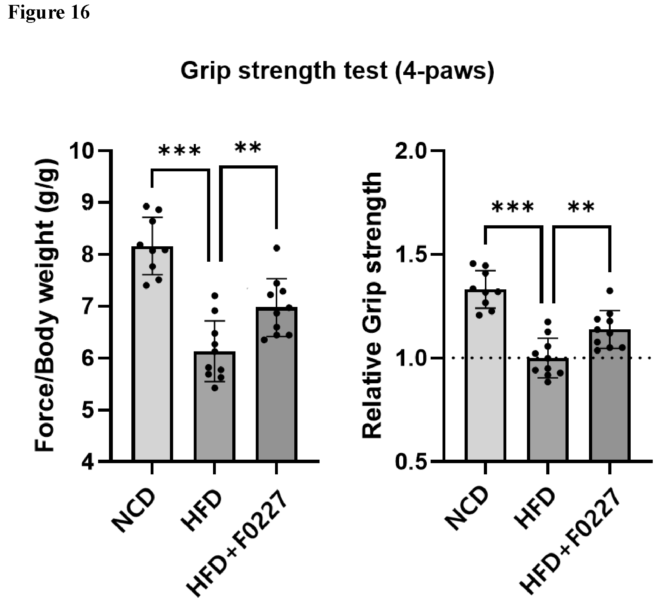

Figure 16 is a graph showing the results of a grip strength test conducted on mice fed the diet shown in Figure 15 for 8 weeks. In the graphs of Figure 16, ** indicates that the p-value calculated by performing a one-way ANOVA test on the corresponding measurement value compared to the reference measurement value is less than 0.01, while *** indicates that the p-value is less than 0.001

### DETAILED DESCRIPTION OF THE INVENTION

Reference will now be made in detail to certain embodiments, examples of which are illustrated herein. As an initial matter, it should be duly noted that the meanings of the terms or words used in this disclosure and/or the claims are not to be restricted to their ordinary or dictionary meanings, but should be construed in agreement with the technical idea of the present invention under the principle that an inventor can act as his own lexicographer.

While working examples will be described herein, it will be understood that they are intended to provide better understanding of the present invention. As these working examples are not meant to constitute and represent the technical idea of the present invention in its entirety, one skilled in the art would recognize that many equivalents or modifications to the working examples are possible and they also fall within the scope of the present invention.

Unless defined otherwise, all technical and scientific terms used herein have the same meaning as is commonly understood by one of skill in the art to which the claimed subject matter belongs. The present disclosure describes exemplar methods and/or materials but similar or equivalent methods and/or materials are also categorically encompassed by the present invention. Any numerical value used in this disclosure is to be understood to include before it, the term "about" even in the absence of the term. Any numerical range used in this disclosure includes both the starting value and the ending value respectively as the minimum and maximum defining the boundaries of the numerical range.

As used herein, the term "metabolic syndrome" is a condition characterized by a group of metabolic risk factors including abdominal obesity, insulin resistance or glucose intolerance, atherogenic dyslipidemia, prothrombotic state, proinflammatory state and hypertension. The Adult Treatment Panel defines metabolic syndrome as present if a patient manifests at least three of the following symptoms:
waist measuring at least 40 inches for men, 35 inches for women;
serum triglyceride levels of at least 150 mg/dL;
HDL cholesterol levels of less than 40 mg/dL in men, less than 50 mg/dL in women;
blood pressure of at least 135/80 mmHg and blood sugar (serum glucose) of at least 110 mg/dL.

It has been estimated that up to 25% of the population in the United States are afflicted with metabolic syndrome. An underlying cause of metabolic syndrome is believed to be insulin resistance wherein the ability of insulin to take in glucose from the blood is attenuated. This causes glucose levels to remain elevated after eating to which the pancreas responds by excreting insulin. If left untreated, metabolic syndrome significantly increases the risk of type II diabetes, cardiovascular disease and other diseases related to plaque buildups in artery walls.

As used herein, the term "diabetes" or "diabetes mellitus" refers to a chronic disease characterized by a relative or absolute deficiency of insulin that results in glucoseintolerance. It is contemplated herein that the term diabetes encompasses all types of diabetes, and includes, for example, type 1 diabetes, type 2 diabetes, and hereditary diabetes, but is not limited thereto. Type 1 diabetes is insulin-dependent and primarily results from destruction of β-cells. As used herein, the term "type 2 diabetes" is insulin-independent diabetes, caused by insulin resistance. Type 2 diabetes is caused by the failure by muscle and adipose tissue to detect increase in insulin levels, or the lack of effective insulin function even when detected.

As used herein, the term "insulin resistance" refers to a state in which the cells are incapable of burning glucose effectively due to the failure of the metabolic system of the organism to respond to insulin to lower the blood glucose level. Severe insulin resistance causes overproduction of insulin in excess of what would have been typical for an adequate insulin response and this leads to hyperinsulinemia, hypertension, or dyslipidemia, as well as heart disease and diabetes mellitus.

As used herein, "insulin resistance syndrome" is a generic term referring to all diseases caused by the aforementioned insulin resistance. Insulin resistance syndrome is characterized by cellular resistance to insulin action, hyperinsulinemia and increase in very low-density lipoprotein (VLDL) and triglyceride, decrease in high density lipoprotein (HDL), hypertension and so forth, and is recognized as a risk factor for cardiovascular diseases and type 2 diabetes.

As used herein, the term "culture" refers to a product obtained by culturing a lactic acid bacterium in a known medium, and this product may include the lactic acid bacterium itself. The medium may be selected from art-known liquid or solid media. For instance, the medium can be MRS liquid medium, GAM liquid medium, MRS agar medium, GAM agar medium, or BL agar medium, but is not limited thereto. As used herein, the term "cell-free culture supernatant" refers to the liquid mixture obtained when bacterial cells are removed from a culture of lactic acid bacteria that had been maintained for a certain amount of time. The cell-free culture supernatant is a liquid mixture comprising such components as metabolites released during the culture of the bacterial strain and extra nutrients. Furthermore, "cell-free culture supernatant" herein is contemplated to encompass concentrates of cell-free culture supernatant obtained immediately after completion of lactic acid bacteria culture as well as the extracts thereof and the fractions thereof. Cell-free culture supernatants suitable for use can be made by letting the culture solution to stand for a certain period of time and taking only the liquid from the upper part and leaving out the precipitate in the lower part, by removing the bacterial cells through filtration, or by centrifuging the culture solution to remove the precipitate in the lower part and taking only the liquid in the upper part. As used herein, the term "lysate" refers to disrupted lactic acid bacteria obtained through such means as enzyme treatment, homogenization and ultrasonication of the lactic acid bacteria. In addition, as used herein, the term "cell extract" refers to a product obtained by extracting lactic acid bacteria with an art-known extraction solvent. Furthermore, as used herein, the term, "live cells" refers to the novel lactic acid bacterium of the present invention itself, and the term "killed cells" refers to lactic acid bacteria sterilized by heating, pressurization or drug treatment, or the like.

As used herein, the term "prevention" refers to any and every act of inhibiting or delaying progression of the symptoms of a disease, for example, a metabolic disease in a host that has been administered with the pharmaceutical composition of the present invention.

As used herein, the term "treatment" refers to any and every act improving or beneficially altering the symptoms of a disease, for example, a metabolic disease in a host that has been administered with the pharmaceutical composition of the present invention.

As used herein, the term "secretion", when used in relation to *Lactiplantibacillus plantarum,* refers to a physical phenomenon in which a *Lactiplantibacillus plantarum* strain discharges such substances as metabolites or signaling compounds outside the cell, for example, into the medium or the body of the host.

Unless explicitly indicated otherwise, the term "3-phenyllactic acid", when used in conjunction with extracellular secretion of substance by *Lactiplantibacillus plantarum,* is intended herein to encompasses not only its unionized neutral molecular form (*i.e., 2-*hydroxy-3-phenylpropanoic acid under the IUPAC nomenclature) but also its charged anionic form or its salt forms *(i.e.,* the 2-hydroxy-3-phenylpropanoate anion or salts of this anion).

Unless explicitly indicated otherwise, the term "α-ketoglutaric acid", when used in conjunction with extracellular secretion of substance by *Lactiplantibacillus plantarum,* is intended herein to encompasses not only its unionized neutral molecular form (*i.e., 2-*oxopentanedioic acid under the IUPAC nomenclature) but also its charged anionic form or its salt forms *(i.e.,* the 2-oxopentanedioate anion or salts of this anion).

As used herein, the term "host" refers to the human being who has been administered with a pharmaceutically effective amount of the *Lactiplantibacillus platarum* strain disclosed herein, a lysate thereof, a cell extract thereof, killed cells thereof, a culture thereof or an extract of a culture thereof.

Unless explicitly indicated otherwise, the term "2-hydroxy-3-methylbutyric acid", when used in conjunction with extracellular secretion of substance by *Lactiplantibacillus plantarum,* is intended herein to encompasses not only its unionized neutral molecular form *(i.e.,* the uncharged molecule 2-hydroxy-3-methylbutanoic acid under the IUPAC nomenclature) but also its charged anionic form or its salt forms *(i.e.,* the 2-hydroxy-3-methylbutyrate anion or salts of this anion).

In an aspect of the present invention is provided the *Lactiplantibacillus plantarum* strain GBCC_F0227 (accession number: KCTC 15450BP). Bacteria of the genus *Lactiplantibacillus* are oxygen-tolerant anaerobes widely distributed in nature. These bacteria are gram positive and classified as lactic acid bacteria. Formerly, the genus *Lactiplantibacillus plantarum* was widely known under the old designation of *Lactobacillus.* Microbes belonging to the genus *Lactiplantibacillus* include *Lactiplantibacillus plantarum* and *Lactiplantibacillus sakei.* The present inventors screened and discovered a novel *Lactiplantibacillus plantarum* strain that have superior activities for stimulating GLP-1 secretion as well as of inhibiting DPP-4 function and designated it *Lactiplantibacillus plantarum* GBCC_F0227. This strain was deposited at Korean Collection for Type Cultures under Korea Research Institute of Bioscience and Biotechnology on May 22, 2023 with an accession number of KCTC 15450BP. Furthermore, this strain is a probiotic strain non-toxic to humans and can be utilized without adverse effects. As used herein, *L. plantarum* GBCC_F0227, *Lactiplantibacillus plantarum* GBCC_F0227 (accession number: KCTC 15450BP) and simply GBCC_F0227 without the bacterial species name can also be used to designate the strain *"Lactiplantibacillus plantarum* GBCC_F0227". In the figures and graphs accompanying the specification, this strain may simply be abbreviated as F0227 or f0227.

Strain GBCC_F0227 of the present invention, a lysate thereof, a cell extract thereof, killed cells thereof, a culture thereof, an extract of a culture thereof or a cell-free culture supernatant thereof has the efficacy to promote the secretion of glucagon-like peptide 1 (GLP-1), a hormone that intensifies energy expenditure and suppress appetite in the host.

Strain GBCC_F0227 of the present invention, a lysate thereof, a cell extract thereof, killed cells thereof, a culture thereof, an extract of a culture thereof or a cell-free culture supernatant thereof has the efficacy to promote gene expression of GLP-1 receptor in the host. More specifically, strain GBCC_F0227 of the present invention has the efficacy to promote gene expression of GLP-1 receptor in the host under diet-induced obesity conditions.

The enzyme dipeptidyl peptidase 4 (DPP-4) is known to degrade GLP-1 secreted from enteroendocrine cells (L cells) within 5 minutes inside the body. Therefore, inhibitory activity against this enzyme would be beneficial for the maintenance and function of GLP-1. Strain GBCC_F0227 of the present invention, thanks to its stimulation of GLP-1 secretion, promotion of GLP-1 receptor gene expression and DPP-4 inhibitory activity, is capable of blocking degradation of secreted GLP-1 and maintaining the activity of GLP-1 for an extended period of time, so that potent GLP-1 effect on the host can be expected.

Strain GBCC_F0227 of the present invention, a lysate thereof, a cell extract thereof, killed cells thereof, a culture thereof, an extract of a culture thereof or a cell-free culture supernatant thereof possesses the efficacy to stimulate the appetite-suppressing neurons of the host. More specifically, strain GBCC_F0227 of the present invention, a lysate thereof, a cell extract thereof, killed cells thereof, a culture thereof, an extract of a culture thereof or a cell-free culture supernatant thereof possesses the efficacy to promote expression of genes capable of suppressing appetite, in particular the genes for pro-opiomelanocortin (POMC) and cocaine- and amphetamine-regulated transcript (CART) in the host under diet-induced obesity conditions.

Strain GBCC_F0227 of the present invention, a lysate thereof, a cell extract thereof, killed cells thereof, a culture thereof, an extract of a culture thereof or a cell-free culture supernatant thereof possesses the efficacy to promote gene expression of adiponectin, which provides protection against fat depostion in the liver under diet-induced obesity conditions. Strain GBCC_F0227 of the present invention, a lysate thereof, a cell extract thereof, killed cells thereof, a culture thereof, an extract of a culture thereof or a cell-free culture supernatant thereof possesses the efficacy to suppress gene expression of CD36 and PPARγ, which genes contributing to visceral triglyceride deposition under diet-induced obesity conditions.

Strain GBCC_F0227 of the present invention, a lysate thereof, a cell extract thereof, killed cells thereof, a culture thereof, an extract of a culture thereof or a cell-free culture supernatant thereof possesses the efficacy to reduce blood triglyceride levels in the host. More specifically, strain GBCC_F0227 of the present invention, a lysate thereof, a cell extract thereof, killed cells thereof, a culture thereof, an extract of a culture thereof or a cell-free culture supernatant thereof possesses the efficacy to alleviate increase in blood triglyceride levels in the host under diet-induced obesity conditions.

Strain GBCC_F0227 of the present invention, a lysate thereof, a cell extract thereof, killed cells thereof, a culture thereof, an extract of a culture thereof or a cell-free culture supernatant thereof possesses the efficacy to alleviate visceral fat deposition in the host. According to an embodiment of the present invention, the inventive GBCC_F0227 strain can suppress fat deposition in visceral white adipose tissue caused by high fat diet. More specifically, strain GBCC_F0227 of the present invention can suppress increases in the area fraction and size of the lipid fraction in visceral white adipose tissue under high fat diet. Most specifically, strain GBCC_F0227 can suppress fat deposition, in particular, white fat deposition in the liver, thereby contributing to prevention and treatment of fatty liver and non-alcoholic fatty liver disease.

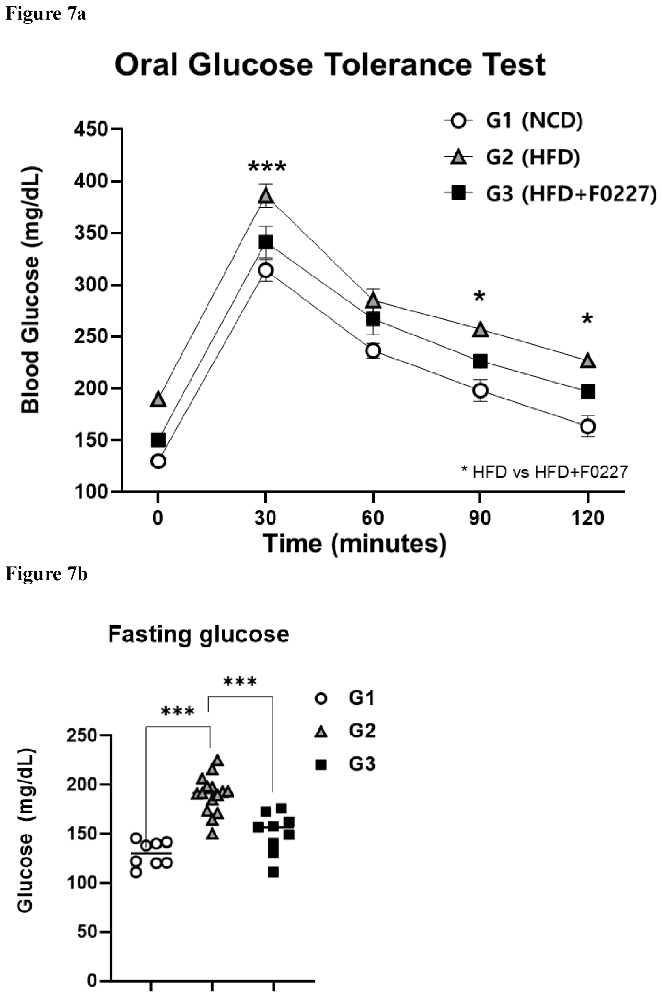

According to an embodiment of the present invention, the aforementioned bacterial strain can reduce blood glucose levels. Preferably, it can reduce fasting blood glucose levels and improve glucose tolerance. Specifically, in an embodiment of the present invention, when the aforementioned bacterial strain was orally administered to a mouse model fed a high fat diet, the fasting blood glucose and post-glucose administration blood glucose levels of the mouse model decreased. Conversely, when a common lactic acid bacterium was orally administered to a mouse model fed a high fat diet, the fasting blood glucose and post-glucose administration blood glucose levels of the mouse model did not decrease. This confirmed that the aforementioned strain reduces fasting blood glucose levels and improves glucose tolerance (Figures 7a to 7c). These effects may lead to an improvement in insulin resistance.

Strain GBCC_F0227 of the present invention has the effect of alleviating the skeletal muscle loss associated with obesity in the host. In particular, strain GBCC_F0227 of the present invention has the effect of alleviating the loss of skeletal muscle mass and/or strength associated with obesity in the host.

Strain GBCC_F0227 of the present invention has the advantageous effect of secreting into the extracellular space large amounts of α-ketoglutaric acid and 3-phenyllactic acid, metabolites that regulate fat metabolism and intestinal flora, and have the function of suppressing weight gain. For example, it secretes larger amounts than the *Lactiplantibacillus plantarum* type strain under the same culture conditions. In one embodiment, the *Lactiplantibacillus plantarum* GBCC_F0227 strain of the present invention secretes 1.5 to 2.5 times more α-ketoglutaric acid and 3-phenyllactic acid than the *Lactiplantibacillus plantarum* type strain KCTC 3108 when cultured in MRS medium. In one specific embodiment, when the *Lactiplantibacillus plantarum* GBCC_F0227 strain of the present invention is cultured in MRS medium for 12 to 16 hours, the secretion amount for each of α-ketoglutaric acid and 3-phenyllactic acid is in the range of 1.5 to 2.5 times the corresponding secretion amount of the *Lactiplantibacillus plantarum* KCTC 3108 strain inoculated and cultured under the same conditions. In a more specific embodiment, when the *Lactiplantibacillus plantarum* GBCC_F0227 strain of the present invention is inoculated into an MRS medium in an amount such that the 600 nm absorbance is 0.01 to 0.02 and cultured for 16 hours, the secretion amount for each of α-ketoglutaric acid and 3-phenyllactic acid is in the range of 1.5 to 2.5 times the corresponding secretion amount of the *Lactiplantibacillus plantarum* KCTC 3108 strain inoculated and cultured under the same conditions. In the most specific embodiment, when the *Lactiplantibacillus plantarum* GBCC_F0227 strain of the present invention is inoculated into MRS medium in an amount such that the 600 nm absorbance is 0.01 to 0.02 and cultured for 16 hours, the secretion amounts of α-ketoglutaric acid and 3-phenyllactic acid are both in the range of 1.95 to 2.05 times compared to the corresponding secretion amounts of the *Lactiplantibacillus plantarum* KCTC 3108 strain inoculated and cultured under the same conditions. In a more specific embodiment, in addition to the above-described secretion characteristics, when the *Lactiplantibacillus plantarum* GBCC_F0227 strain of the present invention is inoculated into an MRS medium in an amount such that the 600 nm absorbance is 0.01 to 0.02 and cultured for 16 hours, the secretion amount of 2-hydroxy-3-methylbutyric acid is in the range of 2.0 to 3.0 times that of the corresponding secretion amount of the *Lactiplantibacillus plantarum* KCTC 3108 strain inoculated and cultured under the same conditions.

Strain GBCC_F0227 of the present invention, a lysate thereof, a cell extract thereof, killed cells thereof, a culture thereof, an extract of a culture thereof or a cell-free culture supernatant thereof exhibits one or more advantageous effects selected from the group consisting of suppressing body weight increase, reducing body fat and suppressing appetite in the host.

In another embodiment, strain GBCC_F0227 of the present invention comprises one or more naturally occurring *Lactiplantibacillus plantarum* mutations.

In another aspect of the present invention is provided a pharmaceutical composition for treating or preventing a metabolic disease, wherein said composition comprises as an active ingredient at least one selected from the group consisting of the *Lactiplantibacillus plantarum* GBCC_F0227 strain, a lysate thereof, a cell extract thereof, killed cells thereof, a culture thereof, an extract of a culture thereof and a cell-free culture supernatant thereof.

The metabolic disease in the pharmaceutical composition according to the present invention for treating or preventing metabolic diseases is one selected from the group consisting of obesity, obesity appetite control, metabolic syndrome, hypertension, atherosclerosis, hyperglycemia, hyperlipidemia, fatty liver, non-alcoholic fatty liver disease, hyperinsulinemia, diabetes, impaired glucose tolerance, hypercholesterolemia, hypertriglyceridemia, cardiovascular disease and insulin resistance syndrome. More specifically, the metabolic disease is one selected from obesity, obesity appetite control, metabolic syndrome, diabetes, impaired glucose tolerance, non-alcoholic fatty liver disease, hypercholesterolemia, hypertriglyceridemia and insulin resistance syndrome. Most specifically, the metabolic disease is obesity, metabolic syndrome, impaired glucose tolerance or diabetes.

The *Lactiplantibacillus plantarum* GBCC_F0227 strain in the pharmaceutical composition according to the present invention is the same as described above. The strain in in the pharmaceutical composition may be live cells or killed cells, preferably live cells. Moreover, the culture of the strain may or may not comprise the strain. Preferably, the culture of the strain comprises the strain.

In an embodiment, strain GBCC_F0227 of the present invention comprises the sequence set forth in SEQ. ID. No.: 2 for the *rec A* gene.

In addition to the active ingredient, the pharmaceutical composition according to the present invention may further include one or more additives selected from the group consisting of preservatives, dyes, emulsifiers, sweeteners, stabilizers, flavor enhancers, flavoring agents, and acidulants.

The pharmaceutical composition of the present invention may further include at least one pharmaceutically acceptable excipient and/or a lyophilizer.

As used herein, the term "pharmaceutically acceptable" with respect to a substance refers to the substance being physiologically acceptable and usually not causing severe gastrointestinal disorders, dizziness, allergic reactions or similar reactions when administered to humans.

The pharmaceutical composition according to the present invention may further comprise at least one pharmaceutically acceptable excipient in addition to the novel lactic acid bacterium. The excipients to be comprised in the composition according to the present invention may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate and mineral oil, and the like, but not limited thereto. The composition of the present invention may be formulated in formulations for oral administration or formulation for parenteral administration using conventional methods, and when formulated, it may be produced using conventional fillers, thickeners, binders, wetting agents, disintegrating agents, surfactants, cryoprotectants, and the like.

For example, the pharmaceutical composition according to the present invention may comprise a cryoprotectant selected from the group consisting of sucrose, maltose, maltodextrin, trehalose, mannitol, sorbitol, inulin, glycerol, DMSO, ethylene glycol, propylene glycol, 2-methyl-2,4-pentanediol, polyethylene glycol, polyvinyl pyrrolidone, polyvinyl alcohol, polyglycerol, skimmed milk, milk protein, whey protein, betaine, adonitol, lactose or any combination thereof.

Preferably, the pharmaceutical composition may comprise as a cryoprotectant, one or more selected from the group consisting of sucrose, skimmed milk and sorbitol. More preferably, the pharmaceutical composition may comprise sucrose, skimmed milk and sorbitol, and specifically, the pharmaceutical composition may comprise 2 to 20% by weight of sucrose, 2 to 20% by weight of sorbitol and 5 to 30% by weight of skimmed milk based on the total weight of the composition. By the addition of the cryoprotectant, the freeze-dried lactic acid bacterium may show significantly increased viability, storage stability, acid resistance and bile resistance. In addition, the addition of antioxidants such as riboflavin, riboflavin phosphate or a physiological acceptable salt thereof, glutathione, ascorbate and cysteine to the freeze-dried composition according to the present invention may further increase the viability of the strain during storage.

When the composition according to the present invention is formulated as a solid preparation for oral administration, the form of the solid formulation includes tablets, pills, powders, granules, capsules and the like. Such a solid preparation may comprise at least one excipient, for example, starch, calcium carbonate, sucrose, lactose or gelatin, or the like, in addition to the active ingredient. In addition, the composition may comprise apart from simple excipients, a lubricant such as magnesium stearate and talc, and the like, but is not limited thereto.

The pharmaceutical composition according to the present invention must be dosed at a pharmaceutically effective amount. As used herein, the term "pharmaceutically effective amount" refers to an amount sufficient for preventing or treating a disease at a reasonable benefit/risk ratio applicable to the medical treatment. The effective dose level may be variously selected by those skilled in the art in consideration of such factors as method of formulation, patient's condition and body weight, patient's gender, age, severity of disease, form of the drug, administration route and period, excretion rate and response sensitivity. The effective dose may be altered according to the route of treatment, use of excipients and the possibility of being used with other drugs, as recognized to those skilled in the art. However, for a preferable effect, in case of oral administration preparations, generally, the composition of the present invention may be administered in an amount of 0.001 to 1000 mg/kg a day, preferably, 0.01 to 100 mg/kg a day, to an adult.

When administering the formulation to administered as described above, the *Lactiplantibacillus plantarum* GBCC_F0227 strain of the present invention may be administered in a dose of 1×10³ colony forming units (CFU)/g to 1×10¹⁶ CFU/g a day. For example, the strain of the present invention may be administered in a dose of 10³ CFU/g to 10¹⁶ CFU/g, 10³ CFU/g to 10¹⁵ CFU/g, 10³ CFU/g to 10¹⁴ CFU/g, 10³ CFU/g to 10¹³ CFU/g, 10³ CFU/g to 10¹² CFU/g, 10⁴ CFU/g to 10¹⁶ CFU/g, 10⁴ CFU/g to 10¹⁵ CFU/g, 10⁴ CFU/g to 10¹⁴ CFU/g, 10⁴ CFU/g to 10¹³ CFU/g, 10⁴ CFU/g to 10¹² CFU/g, 10⁵ CFU/g to 10¹⁶ CFU/g, 10⁵ CFU/g to 10¹⁵ CFU/g, 10⁵ CFU/g to 10¹⁴ CFU/g, 10⁵ CFU/g to 10¹³ CFU/g, 10⁵ CFU/g to 10¹² CFU/g, 10⁶ CFU/g to 10¹³ CFU/g, 10⁶ CFU/g to 10¹² CFU/g, 10⁷ CFU/g to 10¹³ CFU/_{g}, 10⁷ CFU/g to 10¹² CFU/g, 10⁸CFU/g to 10¹³ CFU/g or 10⁸CFU/g to 10¹² CFU/g. Such doses may be administered in a single dose a day or it may be divided into multiple doses a day. The foregoing is not intended to limit the scope of the present invention in any aspect.

When formulated as a liquid preparation for oral administration, the composition of the present invention may comprise such components as suspending agents, oral liquids, emulsifiers and syrup. The composition as liquid preparation may comprise various excipients, *e.g*., wetting agents, sweeteners, flavors and preservative in addition to commonly used simple diluents, such as water and liquid paraffin, but it is not limited thereto. When formulated as a preparation for parenteral administration, the composition of the present invention may comprise sterilized aqueous solution, non-aqueous solvent, suspending agents, emulsifiers and suppositories. For non-aqueous solvent and suspending agent, the composition may comprise propylene glycol, polyethylene glycol, plant oils such as olive oil, injectable esters such as ethyl oleate, and the likes, but it is not limited to the foregoing. As a base compound for suppositories, the present invention may comprise witepsol, macrogol, tween 61, cacao butter, laurinum, glycerogelatin, and the like. The content of the active ingredient, the novel lactic acid bacterium, in the pharmaceutical composition of the present invention may be adjusted within a variety of ranges depending on the specific form of the composition, its intended purpose, or its application. The content of the active ingredient in the pharmaceutical composition according to the present invention is not particularly limited, and may be, for example, 0.01 to 99%, specifically 0.1 to 75%, and more specifically 0.5 to 50% by weight based on the total weight of the composition. The role of cryoprotectant used in the present invention is to preserve the probiotic formulation during freeze-drying and improve its shelf-life. The cryoprotectant used in the present invention may comprise conventional saccharide cryoprotectants. These saccharides may be a mono-, di-, oligo-, or poly-saccharide or a mixture of at least 2 or more of saccharides.

The pharmaceutical composition according to the present invention may be administered via various routes to human beings, mammals and livestock, for example, monkeys, mice, rats, rabbits, sheep, cattle, dogs, horses and pigs. Specifically, the pharmaceutical composition of the present invention may be orally or parenterally administered (for example, applied or injected intravenously, subcutaneously, intraperitoneally). Preferably, it is administered orally. Solid preparations for oral administration may include powders, granules, tablets, capsules, soft capsules, pills, and the like.

The pharmaceutical composition of the present invention may be provided as an enteric coated enteric preparation, in particular in oral unit dose formulations. As used herein, the term "enteric coating" includes all kinds of pharmaceutically acceptable coatings known, which are not decomposed by and maintained in gastric acid, but are sufficiently decomposed in the small intestine to allow active components to be released there. The "enteric coating" of the present invention refers to a coating that remains intact for 2 hours or more when in contact with an artificial gastric juice such as HCl solution of pH 1 at 36° C to 38° C and preferably, is decomposed thereafter within 30 minutes in an artificial intestinal juice such as KH₂PO₄ buffer solution of pH 6.8.

In an embodiment of the pharmaceutical composition according to the present invention, strain GBCC_F0227, a lysate thereof, a cell extract thereof, killed cells thereof, a culture thereof, an extract of a culture thereof or a cell-free culture supernatant thereof comprises one or more selected from the group consisting of α-ketoglutaric acid, 3-phenyllactic acid, cystathionine, 1-monopalmitin, glycerol monostearate and 2-hydroxy-3-methylbutyric acid.

In an embodiment of the pharmaceutical composition according to the present invention, strain GBCC_F0227, a lysate thereof, cell extract thereof, killed cells thereof, a culture thereof, an extract of a culture thereof or a cell-free culture supernatant thereof inhibits DPP-4 enzymatic activity.

In an embodiment of the pharmaceutical composition according to the present invention, strain GBCC_F0227, a lysate thereof, cell extract thereof, killed cells thereof, a culture thereof, an extract of a culture thereof or a cell-free culture supernatant thereof promotes GLP-1 secretion in the host. The pharmaceutical composition according to the present invention under this embodiment possesses the efficacy to inhibit DPP-4 enzymatic activity.

In an embodiment of the pharmaceutical composition according to the present invention, strain GBCC_F0227, a lysate thereof, cell extract thereof, killed cells thereof, a culture thereof, an extract of a culture thereof or a cell-free culture supernatant thereof promotes the host GLP-1 receptor expression. The pharmaceutical composition according to the present invention under this embodiment possesses the efficacy to inhibit DPP-4 enzymatic activity.

In an embodiment of the pharmaceutical composition according to the present invention, strain GBCC_F0227, a lysate thereof, cell extract thereof, killed cells thereof, a culture thereof, an extract of a culture thereof or a cell-free culture supernatant thereof alleviates loss in skeletal muscle mass that accompanies obesity in the host.

In an embodiment of the pharmaceutical composition according to the present invention, strain GBCC_F0227, a lysate thereof, cell extract thereof, killed cells thereof, a culture thereof, an extract of a culture thereof or a cell-free culture supernatant thereof alleviates visceral fat deposition in the host. In particular, strain GBCC_F0227, a lysate thereof, a cell extract thereof, killed cells thereof, a culture thereof, an extract of a culture thereof or a cell-free culture supernatant thereof alleviates liver fat deposition in the host.

In an embodiment of the pharmaceutical composition according to the present invention, strain GBCC_F0227, a lysate thereof, cell extract thereof, killed cells thereof, a culture thereof, an extract of a culture thereof or a cell-free culture supernatant thereof reduces blood triglyceride levels in the host.

In still another aspect of the present invention is provided a pharmaceutical composition for treating or preventing metabolic diseases in which the composition comprises as an active ingredient at least one selected from the group consisting of a strain of the species *Lactiplantibacillus plantarum,* a lysate thereof, a cell extract thereof, killed cells thereof, a culture thereof, an extract of a culture thereof and a cell-free culture supernatant thereof, and the composition increases the GLP-1 secretion or suppresses the appetite of the host.

In an embodiment of the composition mentioned above, the strain of the species *Lactiplantibacillus plantarum,* the lysate thereof, the cell extract thereof, the killed cells thereof, the culture thereof, the extract of a culture thereof or the cell-free culture supernatant thereof possesses one or more effects selected from the group consisting of host body weight increase, reducing host body fat and suppressing appetite in the host.

In a specific embodiment, the *Lactiplantibacillus plantarum* strains suitable for use as the strain of the species *Lactiplantibacillus plantarum* in the pharmaceutical composition for treating or preventing metabolic diseases are those whose genomes have an average nucleotide identity (ANI) to that of the *Lactiplantibacillus plantarum* strain GBCC_F0227 (accession number: KCTC 15450BP) of 69% or more but less than 100%. *Lactiplantibacillus plantarum* strains with an ANI value of 69% or higher but less than 100% relative to the GBCC_F0227 genome can induce host GLP-1 secretion to levels similar to GBCC-F0227, affording them an advantage in enhancing the host's GLP-1 action. In a still more specific embodiment, the *Lactiplantibacillus plantarum* strain for the pharmaceutical composition is a strain with a genome having an ANI value of 69% or higher but less than 100% relative to the GBCC_F0227 genome and this *Lactiplantibacillus plantarum* strain, a lysate thereof, a cell extract thereof, killed cells thereof, a culture thereof, or a cell-free culture supernatant thereof comprises one or more selected from the group consisting of α-ketoglutaric acid, 3-phenyllactic acid, cystathionine, 1-monopalmitin, glycerol monostearate and 2-hydroxy-3-methylbutyric acid.

In a further aspect of the present invention is provided a food composition comprising the *Lactiplantibacillus plantarum* strain GBCC_F0227 (accession number: KCTC 15450BP) or a culture thereof. The *Lactiplantibacillus plantarum* GBCC_F0227 strain in the food composition according to the present invention is the same as described above. All possible forms including functional foods, nutritional supplements, health foods, and food additives are encompassed in the food composition. The food composition according to any of these types can be prepared in various forms using conventional methods known in the art. Moreover, as used in herein, the term "food composition" is intended to encompass not only the aforementioned food given as examples of food composition, but also "food additives" or "food additive compositions" that are incorporated into such food. When the aforementioned strain is used as a food additive, the bacterial strain may be added as it is or may be used together with other foods or food ingredients appropriately by means of conventional methods. The amount of the active ingredient in the product mixture can be appropriately determined according to the purpose of use (prevention, health maintenance or therapeutic treatment). In general, the bacterial strain may be added in an amount of 0.0001% to 1% by weight, specifically 0.001% to 0.1% by weight to the raw material composition when preparing food or beverage. However, in the case of long-term intake for the purpose of health, hygiene or health control, an amount below the above range may also be used. The food composition according to the present invention can be used for the purpose of prevention or treatment of metabolic diseases.

In a still further aspect of the present invention is provided a dietary supplement comprising as an active ingredient at least one selected from the group consisting of the *Lactiplantibacillus plantarum* strain GBCC_F0227 (accession number: KCTC 15450BP), a lysate thereof, cell extract thereof, killed cells thereof, a culture thereof, an extract of a culture thereof and a cell-free culture supernatant thereof. The dietary supplement according to the present invention can be used for the purpose of prevention or improvement of obesity.

As used herein, the term "dietary supplement" refers to a food that uses a specific ingredient as its raw material or food produced and manufactured for the specific ingredient originally present in raw food material using such methods as extraction, concentration, purification or mixing for the purpose of health supplementation. A dietary supplement is food designed and processed to enable the body to sufficiently exert biological regulatory functions such as biological defence, regulation of biological rhythms, and prevention and recovery from disease through this specific ingredient. Dietary supplements may perform functions associated with the prevention and recovery of a disease.

When the *Lactiplantibacillus plantarum* strain GBCC_F0227 according to the present invention is used as a dietary supplement, the bacterial strain may be added as it is or may be used together with other foods or food ingredients, the choice of which can be appropriately made as required. The amount of strain GBCC_F0227 to be added to the product mixture can be appropriately determined according to the purpose of use. The formulation of dietary supplements comprising the *Lactiplantibacillus plantarum* strain GBCC_F0227 according to the present invention is not particularly limited and can be any one of powder, granules, pills, tablets, or capsules as well as general foods or beverages. The food is not limited to particular kinds, and examples of food to which the strain may be added include meat, sausages, bread, chocolates, candies, snacks, confectionery, pizzas, ramens, other noodles, gum, dairy products including ice cream, various soups, beverages, teas, drinks, alcoholic beverages and vitamin complexes. The food encompasses all kinds of food in the usual sense.

As previously described, the *Lactiplantibacillus plantarum* GBCC_F0227 strain of the inventive dietary supplement or this strain used for its culture is a bacterial strain that secretes, when cultured in MRS medium for 12 to 16 hours, α-ketoglutaric acid and 3-phenyllactic acid, both in amounts 1.5 to 2.5 times the corresponding amounts secreted by the *Lactiplantibacillus plantarum* KCTC 3108 strain inoculated and cultured under the same conditions. In a more specific embodiment, the *Lactiplantibacillus plantarum* GBCC_F0227 strain of the inventive dietary supplement or this strain used for its culture is a bacterial strain that secretes, when inoculated into an MRS medium in an amount such that the 600 nm absorbance is 0.01 to 0.02 and cultured for 16 hours, α-ketoglutaric acid and 3-phenyllactic acid, both in amounts 1.5 to 2.5 times the corresponding amounts secreted by the *Lactiplantibacillus plantarum* KCTC 3108 strain inoculated and cultured under the same conditions. In the most specific embodiment, the *Lactiplantibacillus plantarum* GBCC_F0227 strain of the inventive dietary supplement or this strain used for its culture is a bacterial strain that secretes, when inoculated into MRS medium in an amount such that the 600 nm absorbance is 0.01 to 0.02 and cultured for 16 hours, α-ketoglutaric acid and 3-phenyllactic acid, both in amounts 1.95 to 2.05 times the corresponding amounts secreted by the *Lactiplantibacillus plantarum* KCTC 3108 strain inoculated and cultured under the same conditions.. In a more specific embodiment, in addition to the above-described secretion characteristics, the *Lactiplantibacillus plantarum* GBCC_F0227 strain of the inventive dietary supplement or this strain used for its culture is a bacterial strain that secretes, when inoculated into an MRS medium in an amount such that the 600 nm absorbance is 0.01 to 0.02 and cultured for 16 hours, 2-hydroxy-3-methylbutyric acid in an amount 2.0 to 3.0 times that of the corresponding amount secreted by the *Lactiplantibacillus plantarum* KCTC 3108 strain inoculated and cultured under the same conditions.

In a further yet aspect of the present invention is provided a feed composition comprising the *Lactiplantibacillus plantarum* strain GBCC_F0227 (accession number: KCTC 15450BP) or a culture thereof. The *Lactiplantibacillus plantarum* GBCC_F0227 strain in the feed composition according to the present invention is the same as described above. As used in herein, the term "feed composition" is intended to encompass not only the feed itself, but also feed additives (feed additive compositions). The feed composition of the present invention for preventing or ameliorating metabolic diseases can be manufactured by adding strain GBCC_F0227 within an appropriate effective concentration range, in accordance with various feed manufacturing methods known within the feed industry. The inventive feed composition can be used for the purpose of prevention or control of metabolic diseases in livestock.

In a still further yet aspect of the present invention is provided a method for preventing or treating a metabolic disease, the method comprising the step of administrating to a subject the *Lactiplantibacillus plantarum* strain GBCC_F0227 (accession number: KCTC 15450BP) or a culture thereof. The subject may have a metabolic disease. The subject may be a mammal, preferably a human. The *Lactiplantibacillus plantarum* GBCC_F0227 strain in the method according to the present invention is the same as described above. Furthermore, the route of administration, dosage, and frequency of administration of the aforementioned strain or its culture may be varied in method and amount according to the patient's condition and the presence or absence of side effects, and the optimal administration method, dosage, and frequency may be selected within an appropriate range by a person skilled in the art. Moreover, the types of metabolic disorders are as described above.

In yet another aspect of the present invention is provided the *Lactiplantibacillus plantarum* strain GBCC_F0227 (accession number: KCTC 15450BP) or a culture thereof for use in a method for treating a metabolic disease. The *Lactiplantibacillus plantarum* GBCC_F0227 strain is the same as described above. Moreover, the types of metabolic disorders are as described above.

### Examples

The present invention will be described in further detail with reference to the following working and experimental examples. However, it is to be understood that these working and experimental examples are by no means intended to limit the scope of the invention thereto and are provided solely for the purpose of illustrating the present invention. Persons skilled in the art would readily understand that there are available many modifications and alternatives to these examples. All such modifications and alternatives are intended to be encompassed within the scope of the attached claims.

### Preparation Example: Isolation and Identification of Lactic Acid Bacteria Strains from Microbial Sources

Candidate lactic acid bacteria strains were selected from human faecal samples and fermented foods. The fermented foods used as microbial sources were sauerkraut (a German pickled cabbage), napa cabbage kimchi, green onion kimchi, young radish kimchi, winter-grown napa cabbage kimchi and white kimchi. Samples were collected after a specified period of fermentation from sauerkraut (days 9, 12, and 13), napa cabbage kimchi (day 3), young radish kimchi (days 1, 2, 10, and 23), white kimchi (day 5), and winter-grown napa cabbage kimchi (day 3). Samples collected from human faeces and fermented foods were ground in phosphate buffered saline (PBS), serially diluted by 1/10, and plated onto MRS (deMan, Rogosa, Sharpe) plates, which is a lactic acid bacteria culture medium. These media were cultured in an anaerobic chamber at 37°C for more than two days, and bacterial colonies were observed. A single colony was subcultured onto a new MRS plate medium for pure isolation. The pure strain was identified by PCR and sequencing of the 16S rRNA gene. For long-term storage of the strain, 30% (v/v) glycerol was added to the culture broth that reached the end of the exponential growth phase and the cells were stored in a -70°C ultralow temperature freezer. MRS plate and liquid media were also used for cultivation after pure isolation and identification.

Identification of the strains was based on the sequence similarity of the 16S rRNA gene and *recA* gene. DNA was extracted from bacterial cultures using the FastDNA^{®} SPIN Kit for soil (MP Bio, 6560200). Polymerase chain reaction (PCR) was performed on the extracted DNA to obtain genetic information for the identification of these bacteria in which forward primer 27F (5'-AGAGT TTGAT CMTGG CTCAG-3') and reverse primer 1492R (5'-TACGG YTACC TTGTT ACGAC TT-3') were used for the 16S rDNA PCR, and forward primer L. p_recA_F (5'-CTGAT GCACG GAAAG CAGC-3') and reverse primer L. p_recA_R (5'-CGCTT AAATG GYGGY GCMAC C-3') were used for the *recA* PCR.

Using the bacterial genetic information obtained in this manner, we analyzed the strains and identified 324 strains belonging to the genera *Lactiplantibacillus* and *Bifidobacterium.* Specifically, 205 strains of *Lactiplantibacillus plantarum,* 37 strains of *Lactiplantibacillus paracesei,* 45 strains of other *Lactiplantibacillus* bacteria and 45 strains of *Bifidobacterium* were isolated.

These isolates were tested for GLP-1 secretion and inhibition of DPP-4 activity, and superior strains were selected.

### Experimental Example 1: Stimulation of Glucagon-Like Peptide-1 Secretion

To select strains from the aforementioned lactic acid bacteria isolates that stimulate intestinal GLP-1 secretion, the GLP-1 secretion-stimulating properties of the culture supernatants from the isolated strains were evaluated. Among the isolated strains, 114 strains were anaerobically cultured in MRS broth at 37°C for 24 hours. The cells were then precipitated by centrifugation, and only the supernatant was collected. This supernatant was filtered through a 0.22 µm syringe filter to remove cells, yielding the culture supernatant used in the experiments.

The human enteroendocrine cell line NCI-H716 (Korea Cell Line Bank (KCLB) number 10251), known to secrete GLP-1, was seeded at 4 × 10⁴ cells per well in a 96-well plate and cultured overnight. To standardise intercellular GLP-1 secretion levels, the cells were washed three times with DPBS (Dulbecco's phosphate-buffered saline) supplemented with 0.2% bovine serum albumin (BSA) to remove residual media components and incubated for 2 hours. Subsequently, the culture supernatants from various isolated strains were diluted to 20% (v/v) in DPBS supplemented with 0.2% BSA and this dilution was used to treat the NCI-H716 cells for 2 hours. Upon completion of the reaction, the supernatants were collected and analysed using a GLP-1 ELISA kit (Human GLP-1 EIA, Raybiotech) to measure the GLP-1 concentration per strain.

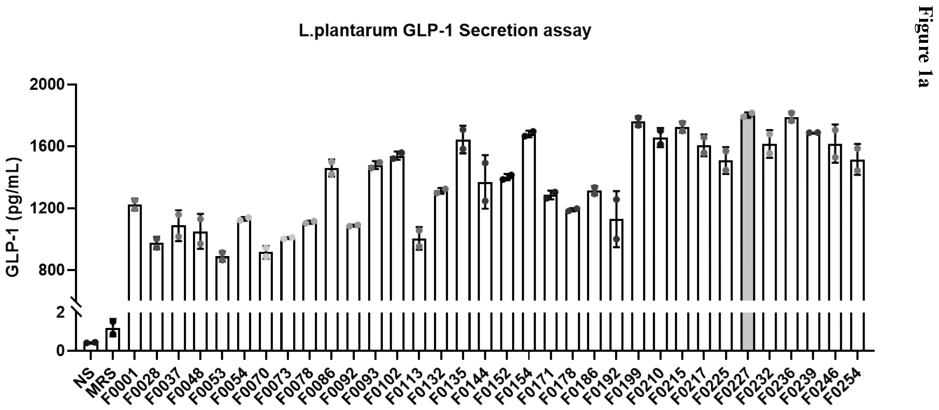

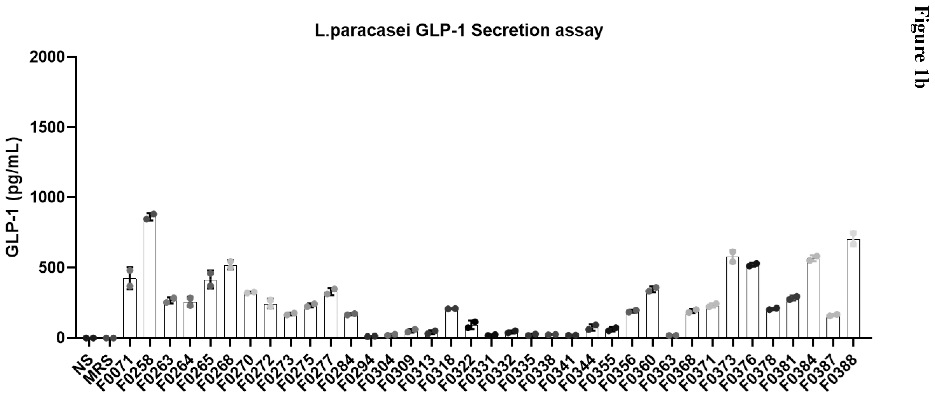

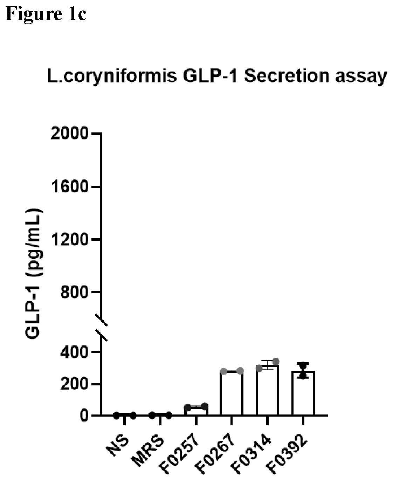

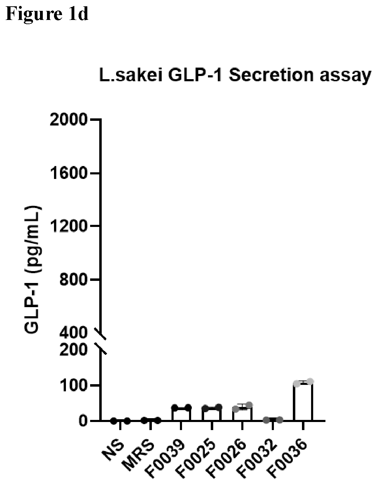

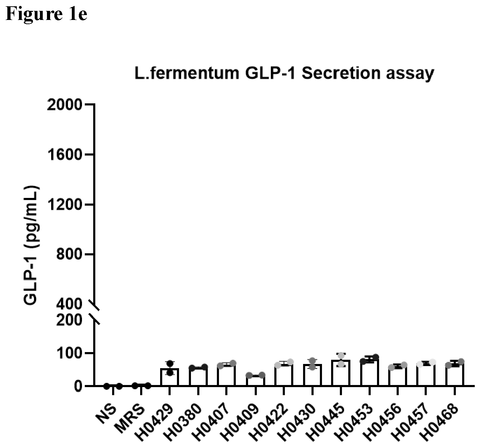

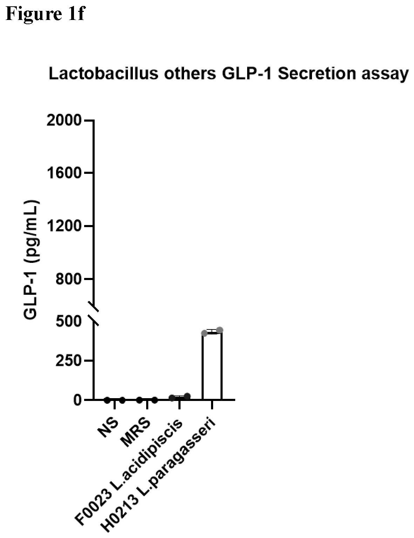

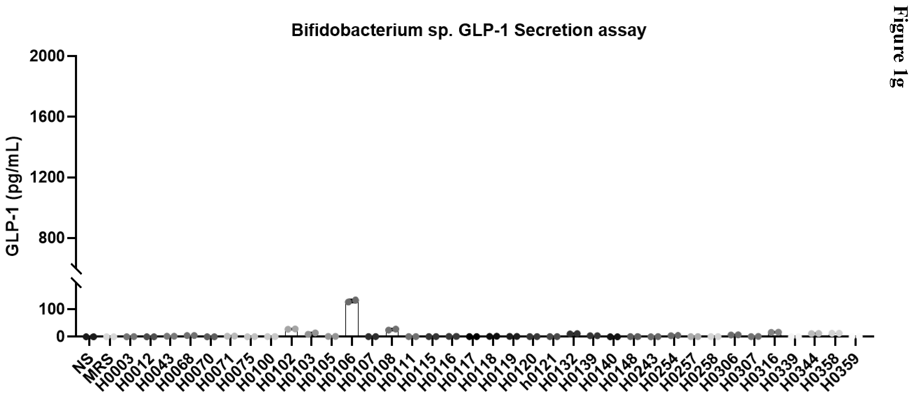

The GLP-1 secretion stimulation performance of the 114 isolated lactic acid bacteria strains are shown in Figures 1a to 1g. As is clearly visible in Figures 1a to 1g, the GLP-1 concentrations in the culture supernatant among the isolated lactic acid bacteria strains were significantly higher for *L. plantarum* strains (Figure 1a) than *L. paracasei* (Figure 1b) or *Bifidobacterium* genus strains (Figure 1g). Furthermore, it was confirmed that *L. plantarum* strain GBCC_F0227, isolated from sauerkraut, exhibited the highest GLP-1 secretion stimulation activity.

### Experimental Example 2: Comparison of Strain-Specific GLP-1 Secretion Stimulation Effects

To determine whether the intestinal GLP-1 secretion stimulatory effect of *L. plantarum* GBCC_F0227, isolated from sauerkraut, was strain-specific, the culture supernatants from KCTC3108, a type strain of the same species provided by the Korea Collection for Type Cultures (KCTC), *L. plantarum* GBCC_F0053, isolated from young radish kimchi, and *L. plantarum* GBCC_F0058, isolated from young radish kimchi, were compared with that of GBCC_F0227 using the same method as in Example 1 for stimulated GLP-1 secretion.

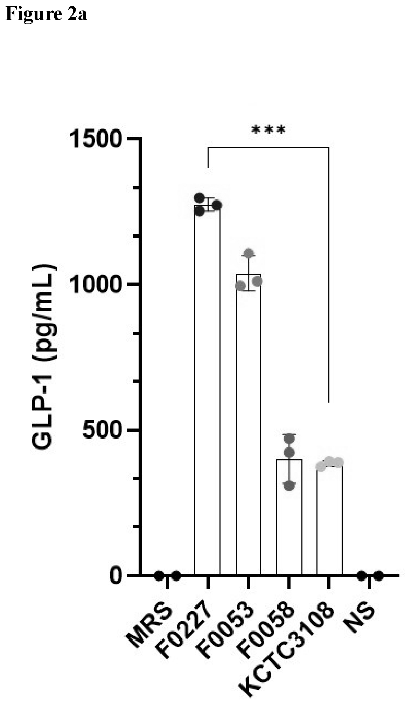

As shown in Figure 2a, the type strain KCTC3108, the same *L. plantarum* strain as GBCC_F0227, as well as other *plantarum* isolates GBCC_F0053 and GBCC_F0058, isolated from sources other than sauerkraut, exhibited GLP-1 secretion-stimulating potency significantly lower than that of GBCC_F0227. This confirms that the superior GLP-1 secretion-stimulating potency of GBCC_F0227 is specific to this strain and is not common to all *L. plantarum.*

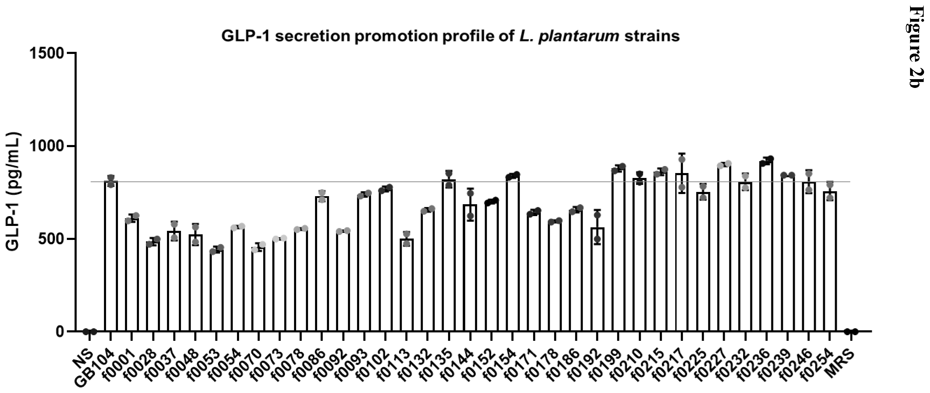

In order to compare the GLP-1 secretion stimulation efficacy of the isolated strains of *plantarum* species, including the F0227 strain, with known *L. plantarum* strains, a comparative experiment was conducted in the same manner as in Example 1 with the known *Lactiplantibacillus plantarum* strain GB104 (Korea Research Institute of Bioscience and Biotechnology Accession No. KCTC15450BP) disclosed in Korean Patent Publication No. 2021-0086540. The experimental results are shown in Figure 2b. Among the isolated strains tested, nine strains, including GBCC_F0227, exhibited better GLP-1 secretion stimulation efficacy than the known *L. plantarum* strain GB104.

### Experimental Example 3: Comparison of the Organism-Level GLP-1 Secretion Stimulatory Effects of F0227

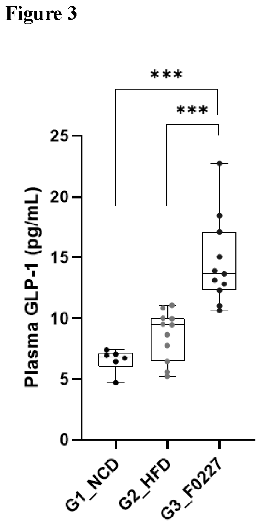

We evaluated whether strain F0227 also stimulates GLP-1 secretion at the organism level. C57BL/6 mice (Orient Bio, Korea) were divided into three groups: the positive control group fed a normal chow diet with no lactic acid bacteria administration, the negative control group fed a high fat diet with 60 caloric percent from fat, and the experimental group administered a high fat diet with concurrent daily administration of 5×10⁹ colony-forming units (CFU) of strain GBCC_F0227. The experiment was conducted for eight weeks, after which the mice were necropsied to determine blood GLP-1 levels. For mice in the high fat diet group, 5×10⁹ colony-forming units (CFU) of *L. plantarum* GBCC_F0227 were orally administered five hours prior to necropsy. Blood was collected through cardiac puncture, and the blood obtained was immediately transferred to a collection tube containing EDTA, to which 500 KIU of aprotinin and 1 µg/mL of diprotin A had been added beforehand and the tubes were stored on ice to prevent biodegradation of GLP-1. The obtained blood was centrifuged to separate out the plasma, and the GLP-1 level in the plasma was measured using a GLP-1 ELISA kit (GLP-1 EIA, Raybiotech) to confirm the efficacy of the strain to raise GLP-1 levels. As a result, it was confirmed that the experimental group (G3_F0227 in Figure 3) administered with GBCC_F0227 together with a high fat diet raised the blood GLP-1 concentration to a significantly higher level than the positive control group (G1_NCD in Figure 3) and the negative control group (G2_HFD in Figure 3).

### Experimental Example 4: Inhibitory Effect of Isolated Strains on Dipeptidyl Peptidase-4 (DPP-4) Activity

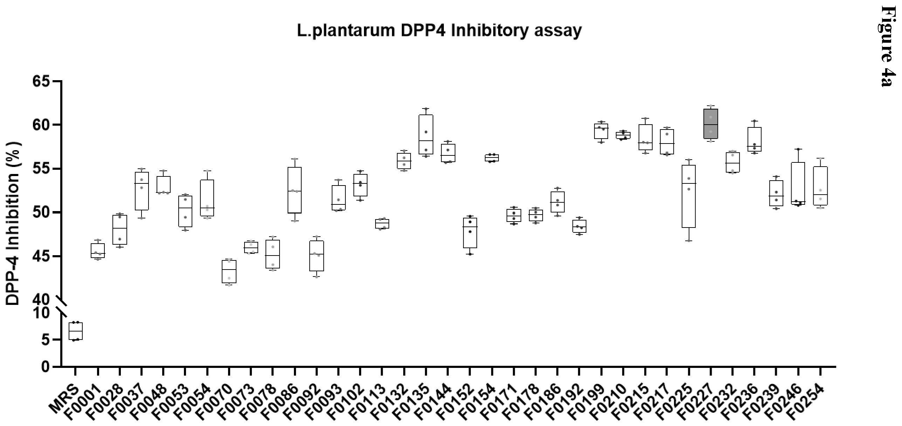

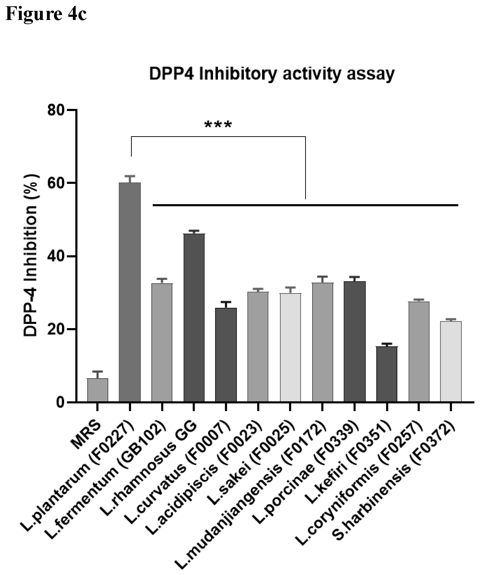

The enzyme DPP-4 is known to degrade GLP-1 secreted from enteroendocrine cells (L cells) within 5 minutes. Therefore, inhibitory activity against this enzyme is considered beneficial for the maintenance and function of GLP-1. To determine whether the culture supernatants of the isolated strains exhibited inhibitory activity against DPP-4, an enzyme inhibition assay was conducted as follows. Gly-Pro-p-nitroanilide (0.8 mM), a substrate for DPP-4, was incubated at 37°C for 10 minutes either in culture supernatants from isolated lactic acid bacteria strains diluted in phosphate buffer (5% v/v) or in MRS medium (5%), a media control, followed by addition of DPP-4 (0.001 enzyme unit) and the enzyme-substrate reaction was allowed to proceed for 60 minutes at 37°C. The reaction was terminated by adding sodium acetate buffer (1 M, pH 4.0) and the decrease in absorbance due to substrate degradation was measured at 405 nm using a microplate reader. The DPP-4 enzyme inhibition of the isolated lactic acid bacteria culture supernatants was evaluated and the results are shown for comparison in Figure 4a. In the experiment shown in Figure 4a, the *Lactiplantibacillus plantarum* GBCC_F0227 strain also exhibited the highest DPP-4 enzyme inhibition among the isolated strains.

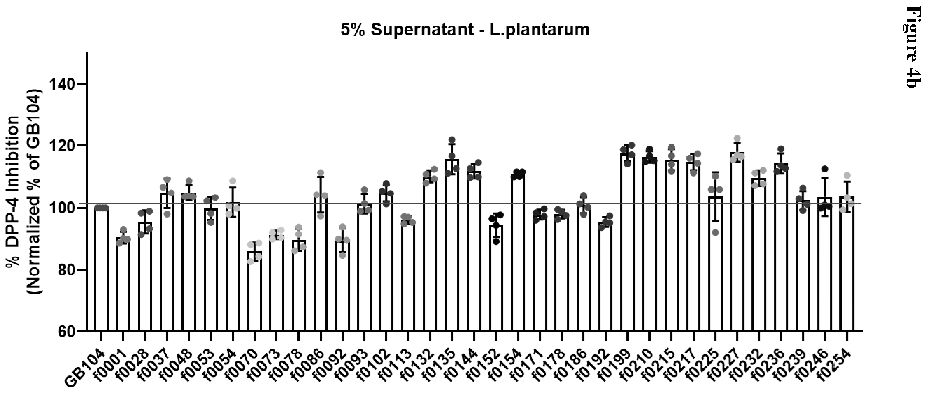

Figure 4b is a graph comparing the DPP-4 activity inhibition efficacies of the selected *Lactiplantibacillus plantarum* strains with that of the known *plantarum* strain GB104. The comparative experiment shown in Figure 4b was performed in the same manner as in Figure 4a. In a comparative analysis where the DPP-4 activity inhibition efficacy of GB104 was set at 100%, GBCC_F0227 showed the highest inhibition efficacy among the isolated strains, with an inhibition efficacy of 117.92%.

### Comparison of Inhibitory Efficacy with Other Lactic Acid Bacteria Species

The DPP-4 inhibitory efficacy of F0227 was compared with those of non-plantarum species of the isolated lactic acid bacteria strains and known *Lactiplantibacillus plantarum* strains. The strains tested for comparison included *Limosilactobacillus fermentum* (GB102) disclosed in Korean Patent Publication No. 2021-0086537, the art-known *Lacticaseibacillus rhamnosus* GG (LGG), *Latilactobacillus curvatus* (GBCC_F0007) isolated from food by the present applicant, *Ligilactobacillus acidipiscis* (GBCC_F0023), *Latilactobacillus sakei* (GBCC_F0025), *Lactiplantibacillus mudanjiangensis* (GBCC_F0172), *Lacticaseibacillus porcinae* (GBCC_F0339), *Lentilactobacillus kefiri* (GBCC_F0351), *Loigolactobacillus coryniformis* (GBCC_F0257), and *Schleiferilactobacillus harbinensis* (GBCC_F0372) as well as *L. plantarum* GBCC_F0227. The culture supernatants obtained after a 16-hour anaerobic incubation for all strains under identical conditions were diluted in phosphate-buffered saline (PBS) to 5% and the DPP-4 activity inhibition was tested as described above. The experimental results shown in Figure 4b indicate that GBCC_F0227 exhibited the highest efficacy with an absolute inhibition rate of approximately 60%, while LGG showed approximately 40% inhibition. All other strains exhibited inhibition rates around 30%, confirming that *L. plantarum* GBCC_F0227 possesses the most potent DPP-4 inhibitory activity.

### Summary of GLP-1 secretion stimulation and DPP-4 inhibition efficacy of L. plantarum isolates

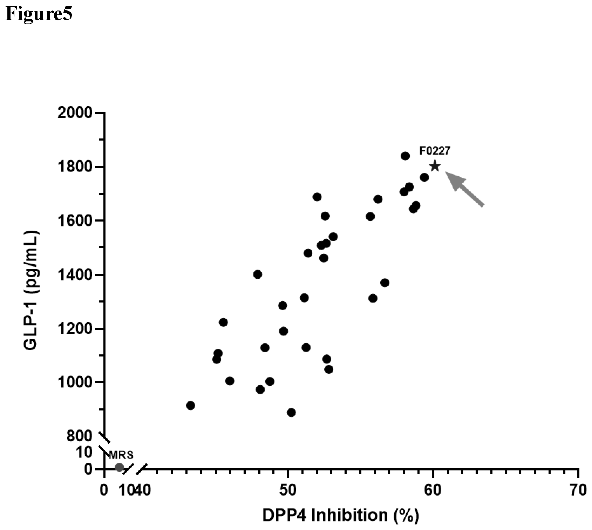

Figure 5 is a graph showing the correlation between the GLP-1 secretion stimulation efficacy and DPP-4 activity inhibition efficacy of GBCC_F0227 and other selected *L*. *plantarum* isolates. Figure 5 demonstrates that GBCC_F0227 stands out in the efficacy evaluation in terms of activities against both GLP-1 and DPP-4 among the selected lactic acid bacteria isolates.

### Experimental Example 5: GBCC_F0227 Gene and Genome Analysis

The full-length 16S rDNA and *recA* gene sequences of GBCC_F0227 are represented by nucleotide sequences designated as SEQ. ID. No.: 1 and SEQ. ID. No.: 2, respectively. Based on the 16S rRNA sequence, GBCC_F0227 showed over 98.5% sequence similarity with 11 type strains of species within the *Lactiplantibacillus* genus, including *Lactiplantibacillus plantarum.* This confirmed that GBCC_F0227 corresponds to one of these 11 strains. The results of the 16S rDNA similarity calculations are summarised in Table 1.

**[Table 1]**

| **Species** | **Type Strain Designation** | **Sequence Similarity (%)** |
|---|---|---|
| *Lactiplantibacillus plantarum* | ATCC 14917(T) | 99.86 |
| *Lactiplantibacillus argentoratensis* | DSM 16365(T) | 99.86 |
| *Lactiplantibacillus pentosus* | DSM 20314(T) | 99.79 |
| *Lactiplantibacillus paraplantarum* | DSM 10667(T) | 99.58 |
| *Lactiplantibacillus daoliensis* | 116-1A(T) | 98.94 |
| *Lactiplantibacillus pingfangensis* | 382-1(T) | 98.94 |
| *Lactiplantibacillus daowaiensis* | 203-3(T) | 98.87 |
| *Lactiplantibacillus nangangensis* | 381-7(T) | 98.87 |
| *Lactiplantibacillus garii* | FI11369(T) | 98.8 |
| *Lactiplantibacillus herbarum* | TCF032-E4(T) | 98.67 |
| *Lactiplantibacillus plajomi* | NB53(T) | 98.67 |

For more precise species identification, similarity analysis was also conducted based on the sequence of the *recA* gene, widely used as a taxonomic biomarker for bacteria classified under the former genus designation *Lactobacillus.* This analysis further confirmed that GBCC_F0227 is most closely related to *Lactiplantibacillus plantarum.* The confirmation of GBCC_F0227 as *Lactiplantibacillus plantarum* was further supported by comparing the average nucleotide identity (ANI) values of the genomes (The exclusion cutoff threshold was set at 95, and the ANI among *Lactiplantibacillus plantarum* strains was 98.78).

**[Table 2]**

| Species | Type Strain Designation | *recA* Sequence Similarity |
|---|---|---|
| *Lactiplantibacillus plantarum* | ATCC 14917 | 100.00% |
| *Lactiplantibacillus argentoratensis* | DSM 16365 | 94.01% |
| *Lactiplantibacillus paraplantarum* | DSM 10667 | 87.94% |

### Genome-based phylogenetic analysis to confirm the intraspecific position of GBCC_F0227

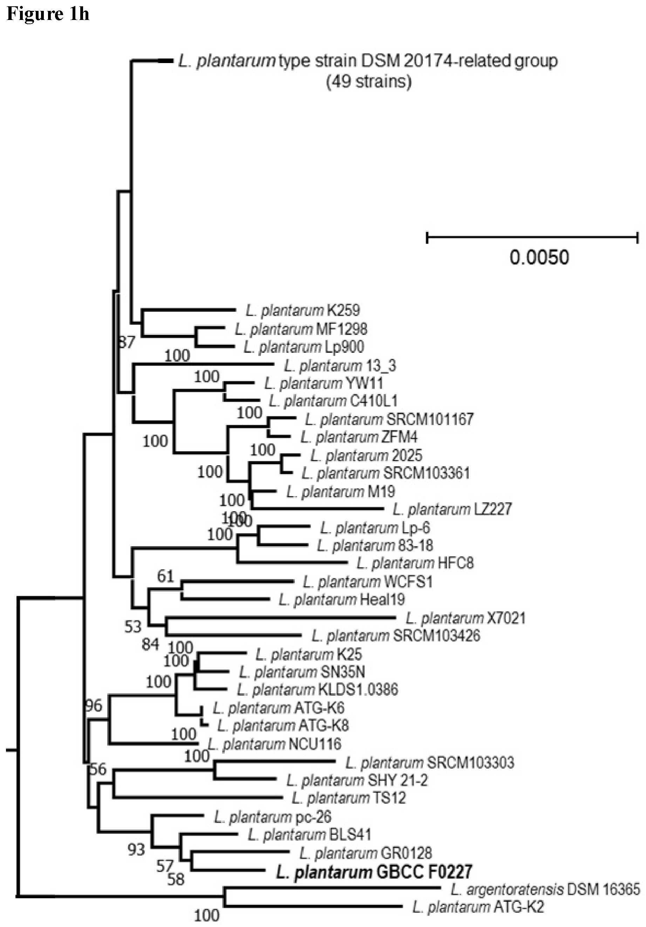

Genome-based phylogenetic analysis was performed to determine the evolutionary relationship of GBCC_F0227 within the *Lactiplantibacillus plantarum* species to which it belongs. The complete genome sequences of 82 *Lactiplantibacillus plantarum* strains published in GenBank, along with one closely related *Lactiplantibacillus argentoratensis* strain, were compared with the GBCC_F0227 genome sequence. The complete genome of *Lactiplantibacillus paraplantarum* DSM 10667^{T} was used as the outgroup for constructing the genome-based phylogenetic tree. Ortholog clustering of protein-coding genes present in the genomes of the strains was performed using the orthoMCL programme. A total of 1,714 genes were selected that were present in all 85 strains' genomes, including the outgroup, and were present only once within each strain's genome. The amino acid sequences of the selected genes were aligned using the MUSCLE alignment algorithm. Based on the aligned amino acid sequences, a phylogenetic tree was constructed using the RAxML programme. The constructed phylogenetic tree is shown in Figure 1h. This tree indicates that the GBCC_F0227 strain originates from a lineage distinct from *Lactiplantibacillus plantarum* type strain DSM 20174^{T} or the existing patented strains ATG-K2, ATG-K6, and ATG-K8.

### Experimental Example 6: Anti-Obesity Effect of F0227

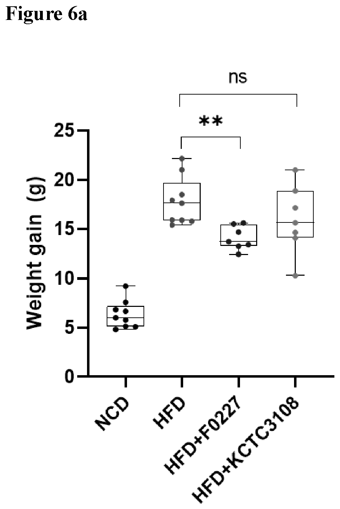

The anti-obesity efficacy of the *L. plantarum* GBCC_F0227 strain was investigated using a high fat diet mouse model. Specifically, C57BL/6 mice were fed a 60% high fat diet (HFD) with concurrent daily oral administration of GBCC_F0227 or the *L. plantarum* type strain KCTC3108. These mice were designated as the experimental groups. Mice fed only the 60% high fat diet served as the negative control group, while mice fed a normal chow diet (NCD) served as the positive control group. GBCC_F0227 and KCTC3108 were administered orally at a daily dose of 5×10⁹ CFU per mouse for 8 weeks. The anti-obesity effect was evaluated by comparing the weight gain in the experimental groups against the negative control. The experimental results shown in Figure 6a demonstrate that when body weight gain at week 8 was compared, the group of mice administered with the *L. plantarum* GBCC_F0227 strain (HFD+F0227 in Figure 6a) had significantly lower weight gain than the KCTC3108-adminstered group (HFD+KCTC3108 in Figure 6a) and the negative control group fed only a high fat diet (HFD in Figure 6a).

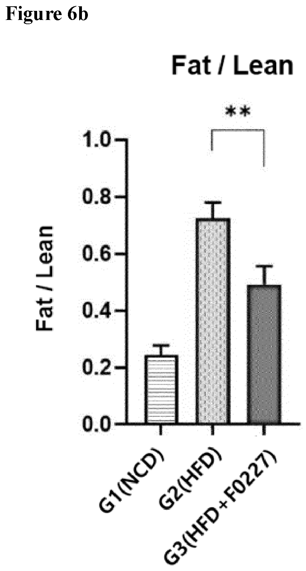

Furthermore, at week 8, a body composition analyzer (LF50 Body Composition Mice Analyzer) was used to measure body fat mass and lean mass and the ratio of body fat to lean mass (Fat/Lean) was compared. As shown in Figure 6b, mice administered with GBCC_F0227 lactic acid bacteria (G3 GBCC_F0227 in Figure 6b) showed a significantly reduced body fat accumulation compared to the negative control mice (G2 HFD in Figure 6b).

### Experimental Example 7: Improved Glucose Control by F0227

The glucose tolerance-improving effect of the *L. plantarum* GBCC_F0227 strain was investigated using an oral glucose tolerance test (OGTT) in a high fat diet-induced obese mouse model. C57BL/6 mice (Orient Bio, Korea) were fed a 60% high fat diet for 8 weeks. One week prior to the end of the animal model experiment, the mice were fasted for 12 hours. Following weight measurement, blood samples were collected from the mouse tail vein at the following time points: immediately before administration in a fasted state, and 30, 60, 90, and 120 minutes after oral administration of glucose at a dose of 2 g/kg. Thereafter, a graph of blood glucose changes was plotted with time (minutes) on the x-axis. The product of blood glucose values and time on the graph was calculated to determine the area under the OGTT curve (AUC).

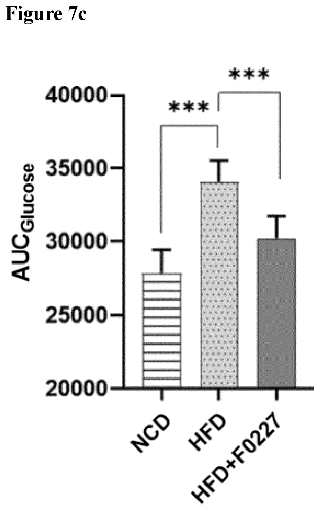

The results of the oral glucose tolerance test are shown in Figure 7a. The group of mice administered *L. plantarum* GBCC_F0227 together with a high fat diet (HFD+F0227 in Figure 7a) showed a significantly lower peak blood glucose level 30 minutes after oral administration of 2 g/kg glucose compared to the negative control group of the high fat diet (HFD in Figure 7a). The measured fasting blood glucose levels shown in Figure 7b also confirmed that the GBCC_F0227-administered group (G3 GBCC_F0227 in Figure 7b) had a significantly lower fasting blood glucose level than the negative control group of the high fat diet (HFD in Figure 7b). The AUC behavior shown in Figure 7c also showed that the GBCC_F0227-administered group mice showed a statistically significant decrease in the AUC value compared to the negative control group. These results confirmed that *L*. *plantarum* GBCC_F0227 reduces fasting blood glucose levels and has a positive effect on improving glucose tolerance.

### Experimental Example 8: Metabolite Analysis of F0227

In this experimental example, the metabolic products that the *L. plantarum* GBCC_F0227 strain secretes outside the cell were analysed and compared with those of other lactic acid bacteria strains. These other lactic acid bacteria strains used for metabolite comparison were *L. platanrum* strains (F0053 isolated from green onion kimchi and KCTC 3108 from a commercially available probiotic lactic acid bacterium), *Limosilactobacillus fermentum* strains (MG901 and KCTC 3112, both commercially available probiotic lactic acid bacteria), a *Lactobacillus gasseri* strain (KCTC 3163, a commercially available probiotic lactic acid bacteria), and a *Lactobacillus paragasseri* strain (M0213, isolated herein) and their metabolites were quantified using gas chromatography-mass spectrometry (GC-MS).

Specifically, single colonies of each lactic acid bacteria strain obtained by culturing on MRS agar medium were inoculated into MRS medium and subjected to seed culture for 24 hours. This seed culture was then inoculated into 50 mL of fresh MRS medium at a cell density corresponding to an absorbance value of 0.01 or 0.02 at 600 nm and incubated at 37°C for 16 hours. The culture was then centrifuged at 8,000 rpm for 5 minutes to obtain the supernatant, which was filtered through a 0.22 µm syringe filter and stored refrigerated until analysis.

200 µL of the culture supernatant from each strain obtained in this manner was subjected to 10 minutes of extraction with cold methanol in a mixer mill (Retch MM400, Germany) operating at 30 Hz, followed by 10 minutes of ultrasonication. Subsequently, the mixture was centrifuged at 12,000 rpm and 4°C for 10 minutes using a centrifuge (Eppendorf 5427R, Germany) to separate the layers. The upper layer was then filtered through a 0.2 µm polytetrafluoroethylene (PTFE) filter (Chromedisk, Korea) and subjected to evaporation using a high-speed vacuum concentrator (Labex, Korea). Each dried sample was then redissolved in 80% methanol to prepare a methanol redissolution solution with a final concentration of 20,000 ppm. 100 µL of this methanol solution from each sample was dried using a high-speed vacuum concentrator and derivatised as follows. First, 50 µL of methoxyamine hydrochloride (20 mg/mL in pyridine solvent) was added to the dried sample and the mixture was placed in a thermal mixer at 30°C for 90 minutes. Next, 50 µL of *N-*methyl-N-(trimethylsilyl)trifluoroacetamide (MSTFA) was added and the mixture was incubated at 37°C for 30 minutes for conversion to a silylated derivative. 1 µL of the silylated derivative of each sample was injected into a CHroZen GC system (Young In Chromas, Anyang, Korea) equipped with an L-PAL3 autosampler system and a YL9600 mass scan spectrum. Metabolites were separated using a DB-5MS column (length 30 m × internal diameter 0.25 mm × particle size 0.25 µm; Agilent Technologies, Santa Clara, USA) with helium as the carrier gas at a flow rate of 1.0 mL per minute. The GC oven temperature was maintained at 60°C for 3 minutes, then increased at a rate of 10°C per minute to reach 320°C, with this final temperature held for a further 3 minutes. The rate of mass spectrometry data acquisition was set at 2750 amu/s over a scan range of 50 - 600 amu, with electron ionisation (EI) mode at -70 eV. The ion source and transfer line temperatures were set to 230°C and 250°C, respectively.

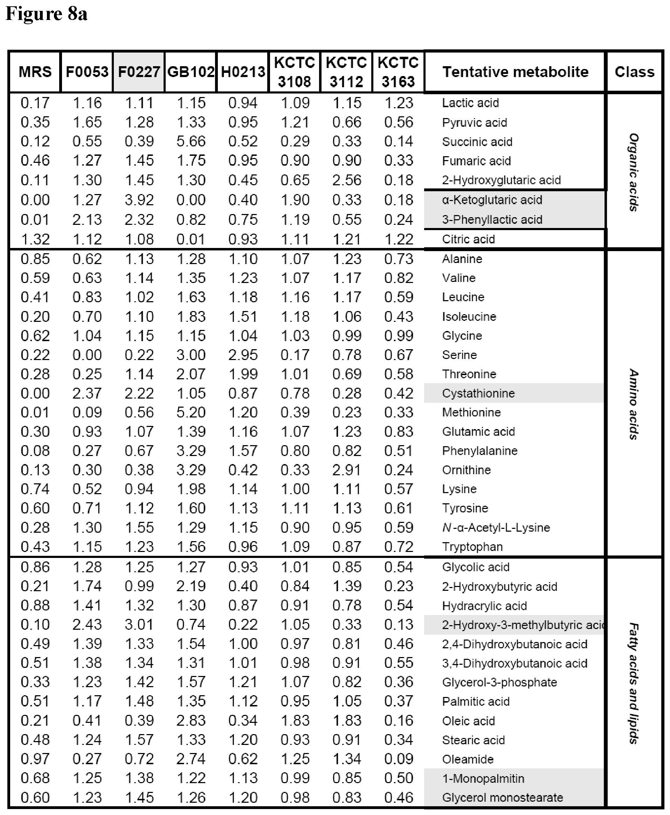

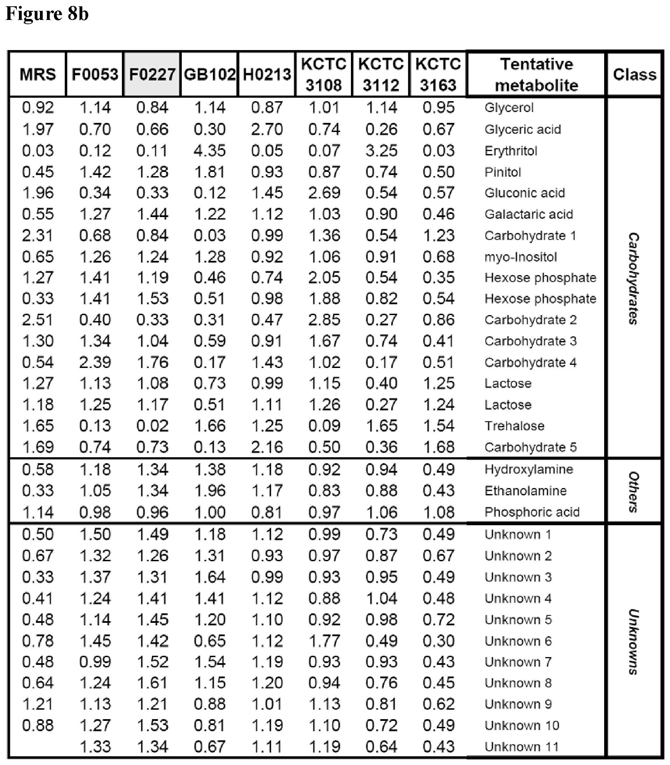

Figure 8 presents a table summarising the heat map, which classifies the quantities of metabolites obtained by GC-MS for each strain by colour, along with their relative numerical ratios. The values indicated in Figure 8 represent, for each metabolite, the ratio of the respective secretion amount for a strain to the average secretion amount averaged across all tested strain samples (peak area of the chromatogram).

The *L. plantarum* GBCC_F0227 strain exhibited a characteristic microbial profile, secreting large quantities of organic acids such as α-ketoglutaric acid and 3-phenyllactic acid, the amino acid cystathionine, and, among lipids and fatty acids, 1-monopalmitin, glycerol monostearate, and the short-chain fatty acid 2-hydroxy-3-methylbutyric acid. Among these, secretion of α-ketoglutaric acid and 3-phenyllactic acid-metabolic products potentially beneficial for preventing weight gain and supporting lipid metabolism in the host-was particularly prominent. The secretion levels of these metabolites reached 1.95 to 2.05 times that of the standard *L. plantarum strain KCTC 3108.* Furthermore, the secretion of 2-hydroxy-3-methylbutyric acid reached a range of 2.0 to 3.0 times that of KCTC 3108.

### Experimental Example 9: Stimulation of GLP-1 secretion by metabolites of F0227

In this example, *in vitro* experiments were conducted to determine whether specific metabolites secreted from the cell by the *L. plantarum* GBCC_F0227 strain possess an intrinsic activity to stimulate GLP-1 secretion. To compare the stimulatory effects of GBCC_F0227 metabolites with those of other lactic acid bacteria, including KCTC 3108, the type strain for *L. plantarum,* the effects of seven metabolites were examined; three metabolites of α-ketoglutaric acid, 3-phenyllactic acid, and 2-hydroxy-3-methylbutyric acid that are abundantly secreted by *L. plantarum* GBCC_F0227, as well as four short-chain fatty acids (SCFAs) of acetic acid, propionic acid, butyric acid, and lactic acid, substances known in the art to stimulate GLP-1 secretion.

Similarly to Experimental Example 1, NCI-H716 cells (KCLB No. 10251) were seeded at 4 × 10⁴ cells per well in a 96-well plate and cultured overnight. To adjust for differences in GLP-1 secretion levels among cells, the cells were washed three times with DPBS supplemented with 0.2% bovine serum albumin (BSA) to remove media components and incubated for 2 hours. The seven substances described above were then diluted in DPBS supplemented with 0.2% BSA to concentrations of 5 mM, 10 mM, 15 mM, 20 mM, or 25 mM, and the NCI-H716 cells were subjected to treatment with these metabolite dilutions for 2 hours. The supernatant collected after the reaction was used to measure the GLP-1 concentration therein using a GLP-1 ELISA kit (Human GLP-1 EIA, Raybiotech).

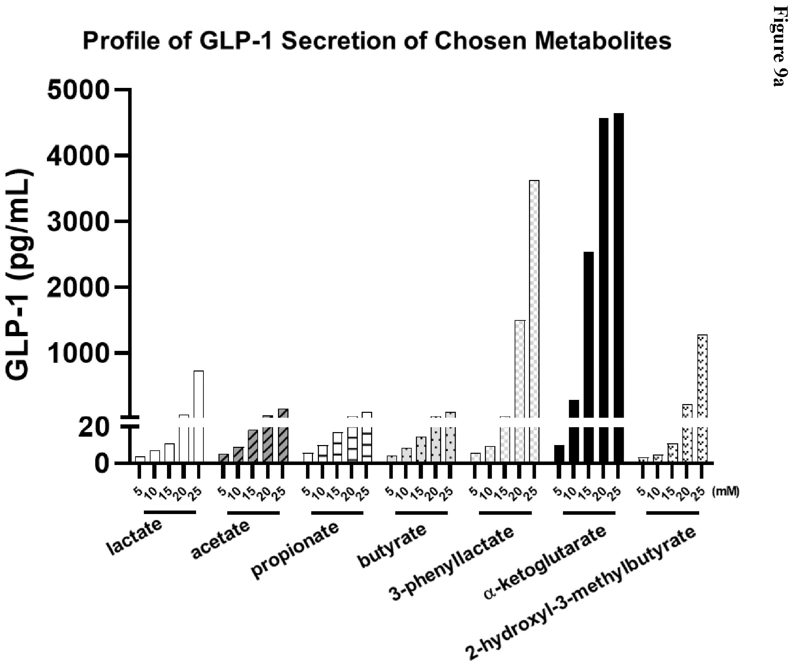

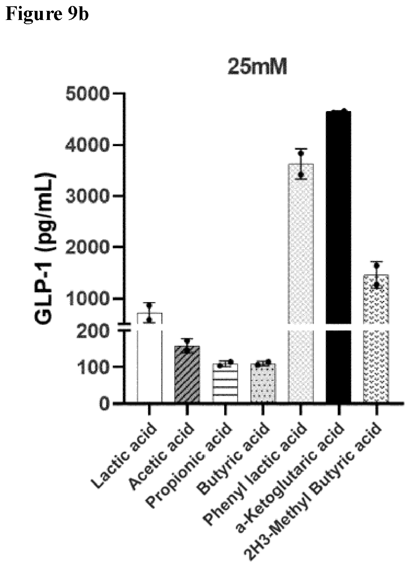

Figures 9a and 9b present the results of this GLP-1 secretion stimulation performance. As shown in Figure 9a, all seven metabolites had the effect of stimulating GLP-1 secretion in NCI-H716 in a dose-dependent manner. Among these, α-ketoglutaric acid and 3-phenyllactic acid, major secreted metabolites of GBCC_F0227, demonstrated secretion efficacy vastly superior to that of short-chain fatty acids, with even lactic acid, the most potent secretion booster among short-chain fatty acids, trailing notably behind 2-hydroxy-3-methylbutyric acid, a metabolite of GBCC_F0227. For instance, the stimulation results at 25 mM metabolite concentrations shown in Figure 9b, α-ketoglutaric acid was shown to have a GLP-1 secretion stimulation effect approximately 46 times more potent than butyric acid, 3-phenyllactic acid approximately 36 times more potent than butyric acid, and 2-hydroxy-3-methylbutyric approximately 12 times times more potent than butyric acid. Furthermore, 3-phenyllactic acid was found to possess a GLP-1 secretion stimulation effect approximately 5 times superior to lactic acid, a common metabolite of lactic acid bacteria, while 2-hydroxy-3-methylbutyric acid was found to possess a GLP-1 secretion stimulation effect approximately 12 times superior to butyric acid. These results indicate that the outstanding human GLP-1 secretion stimulation effect possessed by strain F0227 of the present invention is based on the metabolites of GBCC_F0227.

### Experimental Example 10: Reduction of visceral fat and blood lipids by F0227

This example investigated the *L. plantarum* GBCC_F0227 strain's efficacy in reducing white visceral fat fraction, blood triglycerides and cholesterol levels in a mouse model of obesity induced by a high fat diet. Specifically, five week-old C57BL/6 mice (supplied by Orient Bio, Korea) were acclimatised for one week. They were then divided into three groups and orally administered the following diet daily for eight weeks: the experimental group (G3) fed with a high fat diet (Research Diets, USA, D12492) with 60 kcal% fat content, supplemented daily with 5×10⁹ CFU of *L. plantarum* GBCC_F0227 strain per mouse; the obesity positive control group (G2) receiving the same high fat diet as G3 but administered with cryoprotectant at the same mass as G3's bacterial strain; and the obesity negative control group (G1) receiving a normal chow diet (NCD) (TEKLAD 2018S, Inotiv, USA) supplemented with cryoprotectant.

After 8 weeks, the left lateral lobe of the liver and epididymal fat from the mice were excised via necropsy and were fixed in 10% neutral buffered formalin for 3 days, then subjected to ethanol dehydration, xylene and paraffin infiltration using a tissue processor to be embedded in paraffin. Paraffin sections of 5 µm thickness were mounted on slides and visualised via haematoxylin-eosin staining. Histological quantification of fat fraction in the liver and epididymal fat was performed using ImageJ software. For the left hepatic lobe, the area fraction (%) occupied by lipid droplets within the tissue was quantified. For the epididymal fat, the size (µm²) of lipid droplets accumulated within adipocytes was quantified. Values obtained from three distinct sites per animal were averaged and plotted graphically.

Blood analysis of the mice was performed by obtaining blood via cardiac puncture at necropsy, separating out serum by centrifugation, and measuring total blood cholesterol and triglyceride (neutral fat) levels using an automated clinical chemistry analyser (DRI-CHEM NX700, Fujifilm, Japan).

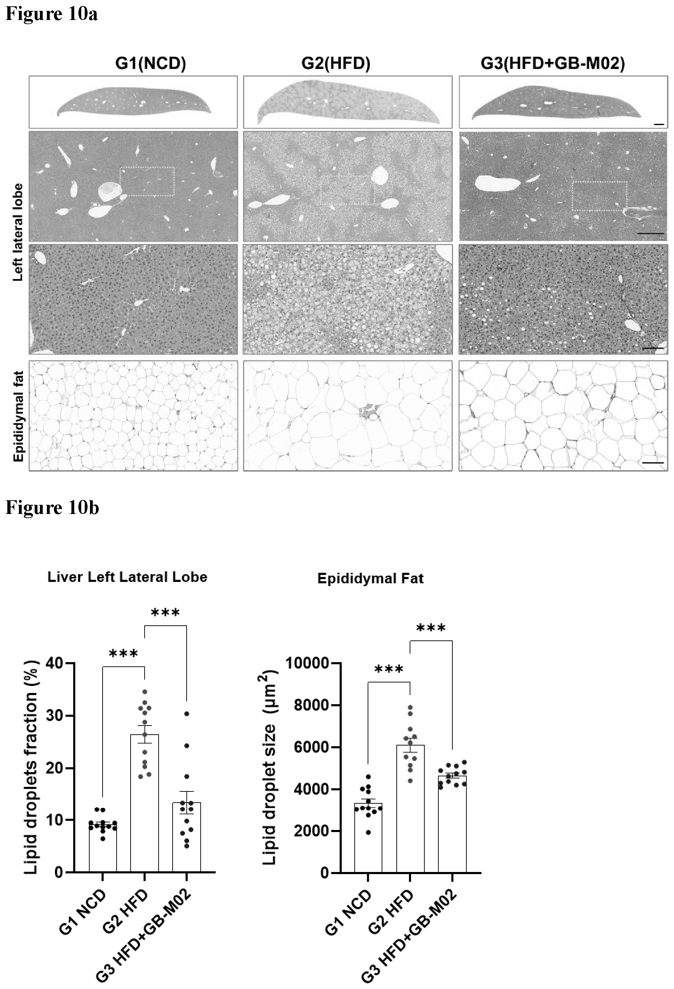

As shown in Figure 10a, the hepatic left lateral lobe tissue sections from high fat diet mice co-administered with *L. plantarum* GBCC_F0227 (G3) exhibited little observable difference to those sections from the obesity negative control group (G1) in terms of morphology and staining patterns of white fat. A marked difference, however, was observed in white fat in comparison with the obesity positive control group (G2) in which obesity was induced by a high fat diet without bacterial strain administration. The bacterial strain-administered group (G3) also showed improvement in fat accumulation in the epididymal fat tissue compared to the obesity positive control group (G2).

The area fraction occupied by lipid droplets within adipocytes in liver tissue and the average size (area) of lipid droplets in epididymal fat tissue also supported these microscopic observations. As shown in Figure 10b, administration of GBCC_F0227 significantly suppressed the increase in lipid fraction caused by the high fat diet, both in terms of area fraction and lipid droplet size.

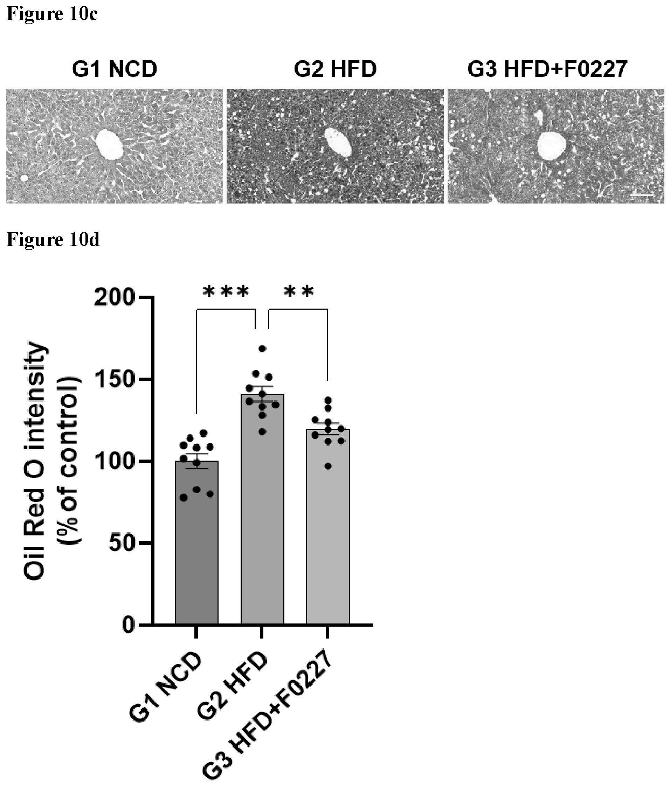

As another method of observing the amount of fat in visceral adipose tissue, oil red O, a reagent specific for neutral fat and fatty acids, was used to stain and visualize fat in the hepatic left lateral lobe tissue sections collected from the three groups above. Fat appears red in Oil Red O staining. Figure 10c shows tissue staining photographs for G1, G2, and G3. The staining results in Figure 10c show that the liver in the normal chow diet (NCD) group (G1) stained close to purple, whereas the liver in the high fat diet group (G2) stained a vivid red, indicating lipid accumulation in the liver tissue due to the high fat diet. In the group receiving both the high fat diet and bacterial strain (G3), the liver tissue was stained a lighter red colour compared to the high fat diet group. This trend in hepatic tissue fat accumulation is better understood by comparing staining results quantitatively. Figure 10d is a graph that compares the staining intensities shown in Figure 10c relative to that of the control group (G1). The staining intensity for the strain-administered group (G3) in Fig 10d was statistically significantly reduced compared to the high fat diet group (G2).

These results indicate that the fat accumulation occurring in the hepatic left lateral lobe due to the high fat diet can be mitigated when the high fat diet is combined with administration of the GBCC_F0227 strain.

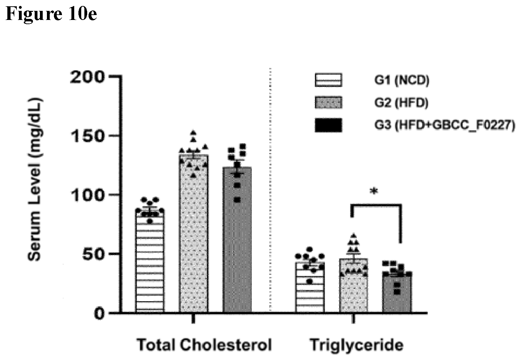

Furthermore, when blood total cholesterol and triglyceride levels are compared, strain GBCC_F0227 of the present invention was effective in reducing the increase in total cholesterol (G3) caused by a high fat diet. Moreover, co-administration of the GBCC_F0227 strain with a high fat diet was shown to significantly bring down the increase in blood triglycerides observed in mice fed a high fat diet alone (G2). The blood triglyceride levels in the co-administration group (G3) were even below those in the normal chow diet (NCD) group (Figure 10e).

Therefore, when strain GBCC_F0227 of the present invention was administered in a high fat diet-induced obese mouse model, it not only exhibited anti-obesity effects in terms of reduced body weight, but also lowered blood triglyceride and cholesterol levels. This confirms the positive effects of strain GBCC_F0227 on various metabolic disease markers, including obesity.

### Experimental Example 11: GBCC_F0227 Strain-Specific Triglyceride Catabolism

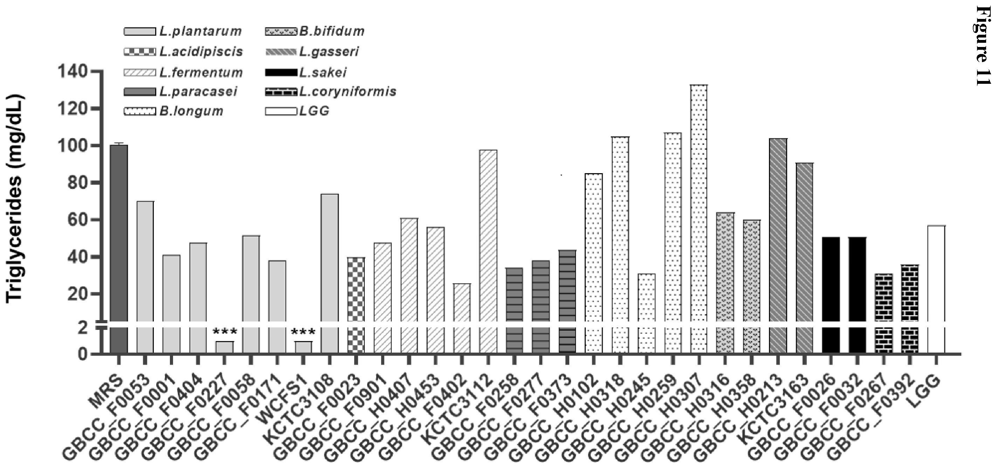

In this example, strains capable of inhibiting the absorption of neutral fat, *i.e.,* triglycerides, were selected and examined for their fat absorption characteristics. Using inhibitory activity against fat absorption as a selection criterion, triglyceride consumption rates before and after incubation were measured for various probiotic bacteria to determine how effectively they catabolized triglycerides present in the culture medium. A total of 31 strains from 10 species were tested, including GBCC_F0227 of the present invention, WCFS1 (a reference strain for *L. plantarum)* and KCTC3108 (a type strain for *L. plantarum*)*,* KCTC3112 (a reference strain for *L. fermentum*)*,* and KCTC3163 (a type strain for *L. gasseri).* The consumption rate test was performed using an automated biochemical analyzer (Clinical Chemistry Analyzer, DRI-CHEM NX700) by comparing the triglyceride content in the culture medium before cultivation with the triglyceride measurements in culture supernatant samples that had been filtered through a 0.22 µm syringe filter after anaerobically culturing the test strains at 37°C in MRS culture medium for 16 hours followed by centrifugation. Figure 11 shows the triglyceride measurements after cultivation for each test strain. Most of the test strains exhibited a decrease in triglyceride content in the culture supernatant after cultivation (the pre-cultivation triglyceride content in the MRS culture medium was approximately 100 mg/dL, see the leftmost gray bar in Figure 11). However, WCFS1 and GBCC_F0227 of the present invention exhibited superior triglyceride consumption compared to the other strains, demonstrating complete catabolisation and consumption of triglyceride in the medium.

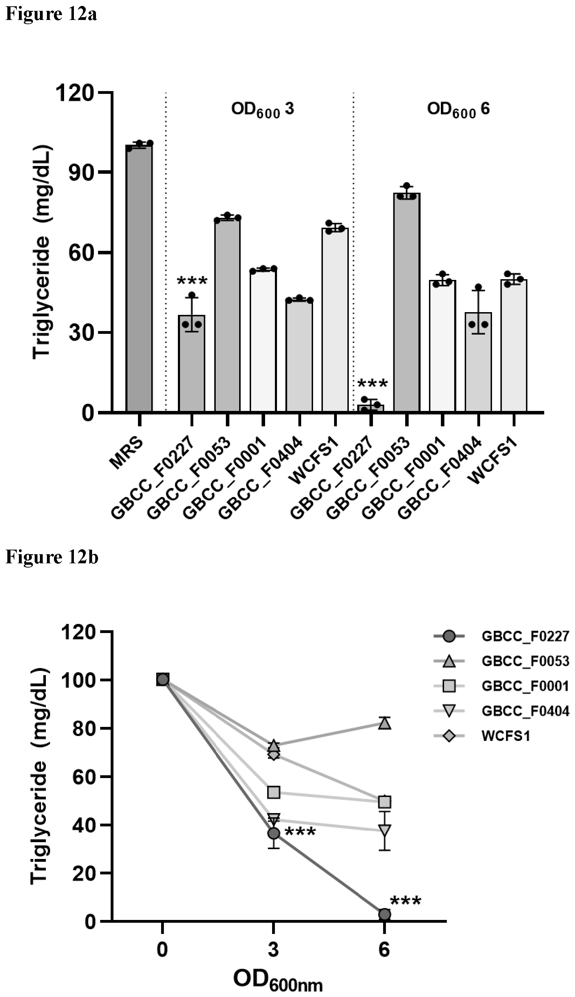

To account for differences among the strains in the extent of metabolism caused by such factors as differing growth rates, which in turn could affect the measured triglyceride consumption rates, another trial of triglyceride measurement was attempted using optical density at 600 nm (OD600) as a surrogate for cell density. In this experiment, one each from four other strains (GBCC_F0053, GBCC_F0001, GBCC_F0404, and WCFS1) belonging to *L. plantarum,* the same species as GBCC_F0227 was cultured under the same conditions until the OD600 reached 3 or 6, and the triglyceride concentration was measured for the supernatant. Similarly to the outcome from the experiment shown in Figure 11, GBCC_F0227 exhibited the most excellent triglyceride catabolism, with the OD600 6 culture, in particular, consuming almost all of the triglyceride at a rate of consumption significantly higher than the other *L. plantarum* strains (Figures 12a and 12b).

### Experimental Example 12: Triglyceride Catabolism Gene Activity of GBCC_F0227

In this example, the superior triglyceride catabolism of GBCC_F0227 of the present invention was verified at the genetic level. The expression of genes belonging to the α/β hydrolase gene family, including lipase, was compared and analyzed for GBCC_F0227 and WCFS1, a strain of the same species *Lactiplantibacillus plantarum* cultured to the same level. Specifically, the two strains, GBCC_F0227 and WCFS1, were anaerobically cultured at 37°C in MRS culture medium to an OD600 level of 3 or 6, and RNA was extracted from the pellet precipitated by centrifugation. cDNA was synthesized using random hexamer primers, and then α/β hydrolysis genes shared by the two strains were selected through genome analysis, and their expression levels were quantified using reverse transcription-quantitative polymerase chain reaction (RT-qPCR). RNA polymerase subunit beta gene (*rpoβ*)*,* a housekeeping gene, was set as a standard for comparing gene expression levels, and Moraxella lipase 2-like (symbol abH04), α/β hydrolase (symbol abH08_1), α/β fold hydrolase (symbol abH08_2), and proline iminopeptidase (symbols abH11_1 and abH11_2) were selected as α/β hydrolase genes shared by the two strains, and their expression was quantified by constructing primers thereof. The relative expression rates of the α/β hydrolase group genes (abH) quantified in this way are shown in Figure 13a (OD600 3) and Figure 13b (OD600 6). GBCC_F0227 showed significantly higher gene expression for abH08_1, abH08_2, and abH11_2 at the cell density of OD600 3 compared to WCFS1 (Figure 13a), and for abH04, abH08_1, abH08_2, and abH1 1_1 at the cell density of OD600 6 (Figure 13b). In addition, the abH08 gene showed a significantly higher expression level than that of WCFS1 in both culture conditions. It was confirmed that abH04 and abH11 exhibited differing increases and decreases in expression levels depending on the culture level conditions. In summary, it was confirmed that GBCC_F0227 can catabolize and consume triglyceride more quickly because the expression of most α/β hydrolase genes was significantly higher in both OD600 3 and 6 conditions than WCFS1.

### Experimental Example 13: F0227's Modulating Effects on Gene Activity Related to Fat Metabolism and Obesity

In this example, it was confirmed that the GBCC_F0227 strain possesses efficacy in regulating the gene activity of proteins related to lipid metabolism and obesity-adiponectin, leptin, PPARγ, CD36, GLP-1 receptor, POMC, and CART-towards anti-obesity in a high fat diet-induced obese mouse model.

Adiponectin is a hormone involved in energy metabolism regulation that lowers blood triglycerides, raises high-density lipoprotein (HDL), and inhibits fat accumulation in the liver. Leptin is an adipokine secreted by adipose cells that regulates food intake and energy expenditure. Blood leptin levels increase in obesity, and it is known in particular that greater severity of non-alcoholic fatty liver disease (NAFLD) correlates with higher blood leptin levels in NAFLD patients. Cluster of differentiation 36 (CD36) is a cell surface receptor that binds to oxidised low-density lipoprotein (LDL) or long-chain fatty acids, and is known to take up free fatty acids into cells, thereby promoting triglyceride accumulation. Peroxisome proliferator-activated receptor gamma (PPARγ) is a nuclear receptor with transcription factor activity, that is present primarily in adipose tissues and is known to induce the transcription of fatty acid uptake-related proteins, including CD36. Pro-opiomelanocortin (POMC) is a hormone precursor synthesised in the pituitary gland. It is cleaved to produce hormones involved in energy metabolism homeostasis and appetite regulation, including ACTH and MSH. Cocaine- and amphetamine-regulated transcript (CART) is a neuropeptide secreted in response to dopamine release, regulating energy metabolism homeostasis and interacting with the hypothalamic appetite control neural circuitry. The GLP-1 receptor is a G protein-coupled receptor that, upon binding its ligand GLP-1, functions to suppress appetite.

Mice were either divided into three groups in the same manner as Experimental Example 10: the experimental group (G3) in which the *L. plantarum* GBCC_F0227 strain is co-administered; the obesity positive control group (G2) in which the same high fat diet was fed along with cryoprotectant in the same amount in weight as the bacterial strain administered in G3; and the obesity negative control group (G1) administered with a normal chow diet (NCD) (TEKLAD 2018S, Inotiv, USA) along with cryoprotectant. Or for some genes, an additional control group (G4) was added, which received a high fat diet and the *L*. *plantarum* type strain KCTC 3108 instead of G3's administered strain but in the same amount. These groups were fed for 8 weeks. After the feeding period, the mice were necropsied, and total RNA was extracted from epididymal white adipose tissue (eWAT), left medial lobe of the liver, and hypothalamic tissue in the brain. Only hypothalamic tissue was collected from the G4 control group. cDNA was synthesized from this RNA using oligodeoxythymidine (Oligo dT), and the expression of adiponectin and leptin genes (epididymis), PPARγ and CD36 genes (left medial lobe of the liver), and POMC and CART genes (hypothalamus) was quantified by RT-qPCR. The housekeeping gene glyceraldehyde-3-phosphate dehydrogenase (GADPH) gene was selected as an internal standard for expression rate quantification, and RT-qPCR was performed using primers designed by referencing the base sequences of genes including adiponectin from the gene database of the National Center for Biotechnology Information (NCBI) in the United States. The expression rate measurement results are summarized in Figures 14a to 14g.

In the epididymis, a visceral white adipose tissue, the expression level of the adiponectin gene in the high fat diet-induced obesity group (G2) dropped significantly to about half the level of normal weight mice in the normal chow diet (NCD) group (G1), whereas in the experimental group (G3) administered with strain GBCC_F0227 of the present invention in conjunction with the high fat diet, the expression of adiponectin rose significantly to a level closely approaching that of the normal chow diet (NCD) group (Fig. 14a). The expression rate of leptin, which significantly increased in the obesity-induced group (G2), also displayed a statistically significant decrease upon administration of strain GBCC_F0227 (Fig. 14b). In the left medial lobe of the liver, the expression of CD36 (Figure 14c) and PPARγ (Figure 14d), which exacerbate hepatic steatosis and fat accumulation, was again observed to increase in the obesity-induced group (G2) compared to the normal chow diet (NCD) group (G1) but administration of GBCC_F0227 caused a statistically significant reduction such that their expression levels were not only restored to levels seen in the normal chow diet (NCD) group (G1) but fell below them.

GBCC_F0227 administration also statistically significantly increased the hypothalamic expression of the GLP-1 receptor (Figure 14e), POMC (Figure 14f), and CART (Figure 14g), which have appetite-suppressing effects, compared to the high fat diet-induced obesity group (G2) or the normal chow diet (NCD) group (G1). Furthermore, the expression rate enhancement effect by GBCC_F0227 was statistically significantly superior to that of the type strain of the same species, KCTC 3108 in the case of the GLP-1 receptor, or statistically comparable for POMC and CD36, with higher average values.

Therefore, it has been demonstrated that administering strain GBCC_F0227 of the present invention in a high fat diet-induced obese mouse model not only produces anti-obesity effects in terms of body weight reduction, but also directs the expression of obesity genes related to fat accumulation, energy metabolism, and appetite towards anti-obesity.

### Experimental Example 14: F0227's Anti-Muscle Mass Loss Effect

This example examined whether GBCC_F0227 possesses efficacy in reversing muscle mass reduction observed in animal models of diet-induced obesity.

Mice were divided into three groups (G1, G2, and G3) in the same manner as in Example 10 and fed for 8 weeks. After the feeding period, the skeletal muscles from the hind limbs-the tibialis anterior (TA), extensor digitorum longus (EDL), soleus (Sol) and gastrocnemius (GA)-were excised and weighed. The measured skeletal muscle weights were normalized to body weight (mg/g) to compare weight differences among the experimental groups. Furthermore, the percentage of muscle mass in each skeletal muscle group was calculated relative to the muscle mass of the high fat diet group (G2).

Experimental results showed that the high fat diet-induced obese mice (G2) exhibited statistically significant loss of muscle mass in all four skeletal muscle groups compared to the normal chow diet (NCD) group (G1). Administration of GBCC_F0227 probiotics statistically significantly restored muscle mass compared to G2. Specifically, compared to G2, GBCC_F0227 administration increased skeletal muscle mass by 1.24-fold in the tibialis anterior (Figure 15a), 1.05-fold in the extensor digitorum longus (Figure 15b), 1.18-fold in the soleus (Figure 15c), and 1.24-fold in the gastrocnemius (Figure 15d).

### Experimental Example 15: Preventive Effect of F0227 on Muscle Strength Loss

This example examined whether GBCC_F0227 possesses efficacy in reversing muscle strength loss observed in animal models of diet-induced obesity.

Mice fed in the same manner as in Example 14 for eight weeks were subjected to a grip strength test on all four paws before dissection. Grip strength tests were performed blindly, with the tester unaware of the experimental group. To prevent exhaustion of the tested mice, measurements were repeated three times per individual at intervals. The average grip strength (in gram-force) was divided by the mouse body weight (g) and expressed as a normalized value (g/g).

Experimental results showed that a marked reduction (p < 0.001) in grip strength compared to the normal chow diet (NCD) group (G1) was associated with the high fat diet-induced obese mice (G2). However, administration of GBCC_F0227 probiotics resulted in a statistically significant recovery of grip strength compared to G2. Specifically, GBCC_F0227 administration increased grip strength in the four paws by 1.13-fold compared to G2, a statistically significant improvement (p < 0.01).

While the foregoing disclosure has described the present invention with reference to a limited number of working examples, it is to be understood that the technical idea and scope of the present invention is not limited to these examples disclosed herein. Embodiments as defined in the appended claims as well as various modifications, changes, and variations to these embodiments that are obvious to one skilled in the art to which the invention pertains are encompassed within the scope of this invention.

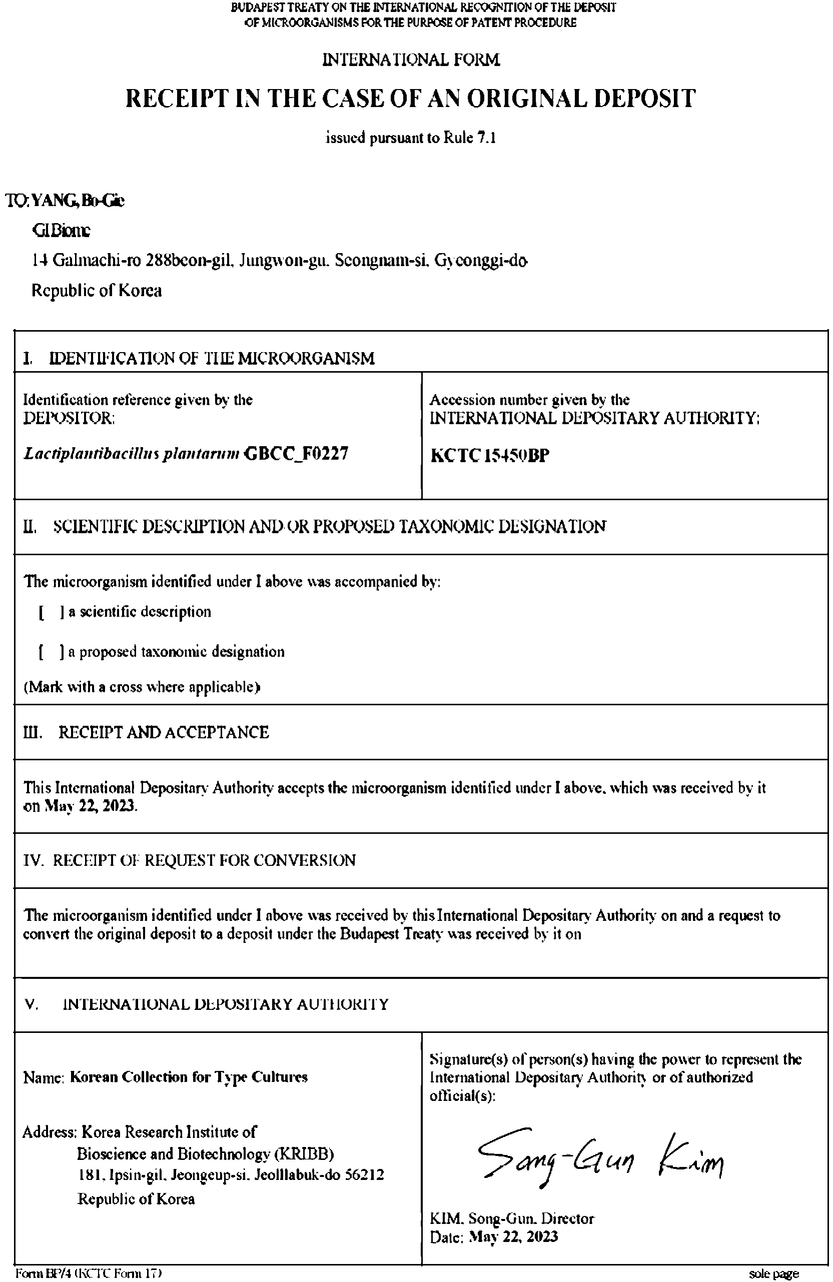
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: YANG, Bo-Gie
GI Biome
14 Galmachi-ro 288beon-gil, Jungwon-gu, Seongnam-si, Gyeonggi-do
Republic of Korea

I. IDENTIFICATION OF THE MICROORGANISM
Identification reference given by the DEPOSITOR: | Accession number given by the INTERNATIONAL DEPOSITARY AUTHORITY:
Lactiplantibacillus plantarum GBCC_F0227 | KCTC 15450BP

II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)

III. RECEIPT AND ACCEPTANCE
This International Depositary Authority accepts the microorganism identified under I above, which was received by it
on May 22, 2023.

IV. RECEIPT OF REQUEST FOR CONVERSION
The microorganism identified under I above was received by this International Depositary Authority on and a request to
convert the original deposit to a deposit under the Budapest Treaty was received by it on

V. INTERNATIONAL DEPOSITARY AUTHORITY
Name: Korean Collection for Type Cultures | Signature(s) of person(s) having the power to represent the International Depositary Authority or of authorized official(s):
Address: Korea Research Institute of
Bioscience and Biotechnology (KRIBB)
181, Ipsin-gil, Jeongeup-si, Jeollabuk-do 56212
Republic of Korea |
KIM, Song-Gun, Director
Date: May 22, 2023

Form BP/4 (KCTC Form 17) sole page

## Claims

1. A pharmaceutical composition for treating or preventing metabolic diseases,
wherein said composition comprises as an active ingredient at least one selected from the group consisting of a strain of the species *Lactiplantibacillus plantarum,* a lysate thereof, a cell extract thereof, killed cells thereof, a culture thereof, an extract of a culture thereof and a cell-free culture supernatant thereof; and
wherein said composition increases the GLP-1 secretion or suppresses the appetite of the host.

2. The composition according to claim 1, wherein the strain of the species *Lactiplantibacillus plantarum,* the lysate thereof, the cell extract thereof, the killed cells thereof, the culture thereof, the extract of a culture thereof or the cell-free culture supernatant thereof has one or more effects selected from the group consisting of suppressing host body weight increase, reducing host body fat and suppressing appetite in the host.

3. *Lactiplantibacillus plantarum* strain GBCC_F0227 (accession number: KCTC 15450BP).

4. The strain of claim 3, wherein the GBCC_F0227 strain has a *recA* gene sequence comprising the nucleotide sequence as set forth in SEQ. ID. No.: 2.

5. The strain of claim 3, wherein the GBCC_F0227 strain comprises one or more naturally occurring *Lactiplantibacillus plantarum* mutations.

6. A pharmaceutical composition for treating or preventing a metabolic disease,
wherein said composition comprises as an active ingredient at least one selected from the group consisting of the *Lactiplantibacillus plantarum* strain GBCC_F0227 (accession number: KCTC 15450BP), a lysate thereof, a cell extract thereof, killed cells thereof, a culture thereof, an extract of a culture thereof and a cell-free culture supernatant thereof.

7. The pharmaceutical composition of claim 6, wherein the metabolic disease is one selected from the group consisting of obesity, obesity appetite control, metabolic syndrome, hypertension, atherosclerosis, hyperglycemia, hyperlipidemia, hypertriglyceridemia, fatty liver, non-alcoholic fatty liver disease, hyperinsulinemia, diabetes, impaired glucose tolerance, hypercholesterolemia, cardiovascular disease and insulin resistance syndrome.

8. The pharmaceutical composition of claim 6, wherein the strain GBCC_F0227, a lysate thereof, a cell extract thereof, killed cells thereof, a culture thereof or an extract of a culture thereof inhibits DPP-4 enzymatic activity.

9. The pharmaceutical composition of claim 6, wherein the strain GBCC_F0227, a lysate thereof, a cell extract thereof, killed cells thereof, a culture thereof, an extract of a culture thereof or a cell-free culture supernatant thereof stimulates GLP-1 secretion in the host.

10. The pharmaceutical composition of claim 6, wherein the strain GBCC_F0227, a lysate thereof, a cell extract thereof, killed cells thereof, a culture thereof, an extract of a culture thereof or a cell-free culture supernatant thereof promotes GLP-1 receptor expression in the host.

11. The pharmaceutical composition of claim 6, wherein the strain GBCC_F0227, a lysate thereof, a cell extract thereof, killed cells thereof, a culture thereof, an extract of a culture thereof or a cell-free culture supernatant thereof stimulates the appetite-suppressing neurons of the host.

12. The pharmaceutical composition of claim 11, wherein the strain GBCC_F0227, a lysate thereof, a cell extract thereof, killed cells thereof, a culture thereof, an extract of a culture thereof or a cell-free culture supernatant thereof stimulates expression of pro-opiomelanocortin or cocaine- and amphetamine-regulated transcript (CART) of the host.

13. The pharmaceutical composition of claim 6, wherein the strain GBCC_F0227, a lysate thereof, a cell extract thereof, killed cells thereof, a culture thereof, an extract of a culture thereof or a cell-free culture supernatant thereof exhibits one or more advantageous effects selected from the group consisting of suppressing host body weight increase, reducing host body fat and suppressing appetite in the host.

14. The pharmaceutical composition of claim 9 or claim 10, wherein the strain GBCC_F0227, a lysate thereof, cell extract thereof, killed cells thereof, a culture thereof, an extract of a culture thereof or a cell-free culture supernatant thereof inhibits DPP-4 enzymatic activity.

15. The pharmaceutical composition of claim 6, wherein the strain GBCC_F0227, a lysate thereof, a cell extract thereof, killed cells thereof, a culture thereof, an extract of a culture thereof or a cell-free culture supernatant thereof alleviates loss in skeletal muscle mass and/or skeletal muscle strength that accompanies obesity in the host.

16. The pharmaceutical composition of claim 6, wherein the strain GBCC_F0227, a lysate thereof, a cell extract thereof, killed cells thereof, a culture thereof, an extract of a culture thereof or a cell-free culture supernatant thereof alleviates visceral fat deposition in the host.

17. The pharmaceutical composition of claim 16, wherein the strain GBCC_F0227, a lysate thereof, a cell extract thereof, killed cells thereof, a culture thereof, an extract of a culture thereof or a cell-free culture supernatant thereof alleviates liver fat deposition in the host.

18. The pharmaceutical composition of claim 6, wherein the strain GBCC_F0227, a lysate thereof, a cell extract thereof, killed cells thereof, a culture thereof, an extract of a culture thereof or a cell-free culture supernatant thereof reduces blood triglyceride levels in the host.

19. The pharmaceutical composition of claim 6, wherein the strain GBCC_F0227, a lysate thereof, a cell extract thereof, killed cells thereof, a culture thereof, an extract of a culture thereof or a cell-free culture supernatant thereof comprises one or more selected from the group consisting of α-ketoglutaric acid, 3-phenyllactic acid, cystathionine, 1-monopalmitin, glycerol monostearate and 2-hydroxy-3-methylbutyric acid.

20. A food composition comprising the *Lactiplantibacillus plantarum* strain GBCC_F0227 (accession number: KCTC 15450BP) or a culture thereof.

21. A dietary supplement comprising the *Lactiplantibacillus plantarum* strain GBCC_F0227 (accession number: KCTC 15450BP) or a culture thereof.

22. A feed composition comprising the *Lactiplantibacillus plantarum* strain GBCC_F0227 (accession number: KCTC 15450BP) or a culture thereof.
